# EUROPEAN PATENT SPECIFICATION

(11) **EP 3 441 764 B1**
(45) Date of publication and mention of the grant of the patent: **27.01.2021**
(21) Application number: 17779052.4
(22) Date of filing: 31.03.2017
(51) Int. Cl.: G01N 33/50, G01N 33/49, G01N 33/68

(54) **METHOD FOR DETERMINING STAGE OF PERIODONTAL DISEASE**
VERFAHREN ZUR BESTIMMUNG DES STADIUMS EINER PARODONTALERKRANKUNG
PROCÉDÉ DE DÉTERMINATION DE STADE DE MALADIE PARODONTALE

(30) Priority: 05.04.2016 JP 2016075980
(43) Date of publication of application: 13.02.2019
(73) Proprietor: ARKRAY, Inc., Kyoto-shi, Kyoto 601-8045 (JP)
(72) Inventor: MAKI, Riichi, Tokyo 130-8644 (JP); FUJIKAWA, Haruhiko, Tokyo 130-8644 (JP); FUKUTA, Isao, Kyoto-shi Kyoto 602-0008 (JP)
(74) Representative: Dehns
(86) International application number: PCT/JP2017/013551
(87) International publication number: WO 2017/175673

(56) References cited:
- EP-A1- 3 156 795
- WO-A1-95/12124
- WO-A1-2012/090995
- JP-A- 2002 253 584
- JP-A- 2005 201 768
- JP-A- 2008 520 366
- JP-A- 2010 256 190
- JP-A- 2013 184 971
- JP-A- 2015 529 333
- JP-A- 2016 001 129
- JP-B2- H 074 273
- US-A1- 2015 038 350
- US-A1- 2016 055 296
- YUUKI KENJI ET AL: "Establishment of Simultaneous Measurement Method of 8 Salivary Components using Urinary Test Paper and Clinical Evaluation of Oral Environment", NIHON HOTETSU SHIKA GAKKAI ZASSHI - JOURNAL OF THE JAPANPROSTHODONTIC SOCIETY, vol. 52, no. 3, 1 July 2008 (2008-07-01), pages 340-349, XP55626977, JP ISSN: 0389-5386
- CHIYOKO UCHIYAMA: "Daeki ni yoru Sogoteki na Koku Kensaho no Kaihatsu (Dai 3 Ho) Takomoku Daeki Kensa System (AL-55) no Shishubyo Kensa Komoku no Monitoring Yuyosei ni Tsuite", Nihon-Shish by -Gakkai-kaishi [Journal of the Japanese Society of Periodontology], vol. 57, 12 September 2014 (2014-09-12), page 123, XP009515885, ISSN: 0385-0110
- EIJI NISHINAGA: "Daeki ni yoru Sogoteki na Koku Kensaho no Kaihatsu (Dai 2 Ho) Takomoku Daeki Kensa System (AL-55) no Kokunai Shojo no Suii ni Taisuru Monitoring Yuyosei ni Tsuite", NIPPON SHIKA NINGEN DOCK GAKKAI SOKAI-GAKUJUTSU TAIKAI [The 16th Academic Conference of Japan Dental Nursing Dock Society]; 13-14/12/2013, vol. 16 th, 2013, page 19, XP009515886,
- EIJI NISHINAGA: "Development of Comprehensive Salivary Test System: -Efficiency of a Newly- developed Salivary Multi-test System (AL-55)", Nihon Shika Hozongaku Zasshi, vol. 58, no. 3, 30 June 2015 (2015-06-30), pages 219-228, XP055528106,
- RIICHI MAKI: "Daeki ni yoru Sogoteki na Koku Kensaho no Kaihatsu (Dai 2 Ho) Takomoku Daeki Kensa System (AL-55) no Shishubyo Kensa Komoku no Yuyosei ni Tsuite", Program and Abstracts of Annual Meeting of the Japanese Society of Periodontology, vol. 56, 8 April 2013 (2013-04-08), page 107, XP009515884, ISSN: 0385-0110
- DAVIS, IAN J: "A cross-sectional survey of bacterial species in plaque from client owned dogs with healthy gingiva, gingivitis or mild periodontitis", PLoS One, vol. 8, no. 12, 2013, page e83158, XP055431181,
- RAESTE, A M: "Rate of migration of oral leukocytes in patients with periodontitis", Scand J Dent Res, vol. 86, no. 1, January 1978 (1978-01), pages 43-51, XP055431184,
- Tohichi Maki: "O-27 Takomoku Daeki Kensa System (AL- 55) no Shishubyo Screening Kensa toshite no Jitsuyosei Kento = [Periodontal disease screen of multi-item saliva examination system (AL-55) Examination as practicality inspection]", Journal of the Japanese Society of Periodontology, vol. 58, no. Suppl. 1, 18 April 2016 (2016-04-18), pages 122-122, XP009514050, ISSN: 0385-0110

## Description

### Technical Field

The present invention relates to an *in vitro* method for determining the stage of a periodontal disease. In addition, the present invention relates to a device, a method, and a program for generating a determination result sheet for presenting a determination result obtained by the determination method.

### Background Art

For the prevention and treatment, it is important to grasp the susceptibility to and the degree of progress of an oral cavity disease. Examples of the oral cavity disease include caries (decayed tooth) and a periodontal disease. Conventionally, the diagnosis of the susceptibility to and the degree of progress of the oral cavity disease has been performed using as a sample saliva or gargled water collected from a subject on the basis of a measurement result of examination items, such as various components and properties, which reflect the susceptibility and the degree of progress.

For example, as the examination items that reflect the susceptibility to and the degree of progress of the periodontal disease, occult blood, white blood cells, alkaline phosphatase, and the like are known. More specifically, when gum tissues are broken in association with the periodontal disease, occult blood is detected in saliva, and white blood cells gather around a diseased area of the periodontal disease. In addition, much alkaline phosphatase is produced from bacteria related to the periodontal disease.

In addition, in a probing examination in which an oral cavity is examined and a probe is inserted into a periodontal pocket, a dentist or the like observes and uses as information for determining the degree of progress of the periodontal disease a periodontal pocket depth and bleeding on probing.

As an examination system regarding periodontal disease bacteria, which has been already put into practical use, there is a system in which, by collecting blood at home and sending the blood in a dedicated container by mail, plasma antibody titers of four types of periodontal disease bacteria are measured, and the degree of infection and an infection type of the periodontal disease bacteria are evaluated. However, the degree of progress (stage) of an actual disease state is not evaluated in this system. In addition, six business days are required until the result is available.

In addition, a system capable of determining the presence or absence of a periodontal disease by detecting occult blood in saliva has also been put into practical use. According to the system, a short-time examination, for example, five minutes, is possible, but an item other than the occult blood cannot be examined, and the degree of progress of the periodontal disease is also not evaluated.

In recent years, a method in which parameters that reflect a caries risk, a periodontal disease risk, and oral cavity cleanliness are measured for saliva obtained from an oral cavity, and risk levels of the caries risk, the periodontal disease risk, and the oral cavity cleanliness are determined from the measurement result has been proposed (PTL 1). According to the method, risk levels, for example, the susceptibility to and the degree of progress of an oral cavity disease, such as a periodontal disease, can be comprehensively determined from the measurement result of multiple examination items. PTL 2 also teaches assessment of a number of different parameters to determine, for example, periodontal disease risk. However, the stage of disease is not determined. PTL 3 similarly assesses intraoral disease risk (such as periodontal disease risk) by measuring individual components or properties in an oral sample but fails to classify the stage of the disease. Kenji et al., 2008, assesses a number of parameters to determine their correlation to periodontal conditions as assessed by dental examination. Eight salivary components are proposed to diagnose periodontal disease, but no classification of the stage of disease is performed.

### Citation List

Patent Literature and published articles

PTL 1: WO 2012/090995 A1
PTL 2: US2015/038350
PTL 3: EP3156795

Kenji et al., 2008, Journal of the Japan Prosthodontic Society, Vol. 52, No. 3, p340-439.

### Summary of Invention

### Technical Problem

All the conventional evaluation method and system of a periodontal disease, including the method described in PTL 1, are determined by a dentist or the like on the basis of measurement values of the examination items, and more specifically, are not established without intervention of a person with professional knowledge and experience.

However, a demand for grasping the susceptibility to and the degree of progress of a personal periodontal disease more easily in a short time is high. Thus, a method capable of objectively, easily, and highly reliably determining the susceptibility to and the degree of progress of a periodontal disease without intervention of a dentist or the like is desired.

In view of these situations, it is an object of the present invention to provide a method for objectively, easily, and highly reliably determining the susceptibility to and the degree of progress (hereinafter, referred to as a "stage") of a periodontal disease without intervention of a dentist or the like on the basis of a measurement result of examination items. In addition, it is also an object of the present invention to provide a device, a method, and a program for generating a determination result sheet for presenting a determination result obtained by the determination method.

### Solution to Problem

In order to solve the above-described problem, the present inventors made extensive research, found that, when determining a stage of a periodontal disease by using as an index the amount of occult blood, the number of white blood cells, a total protein concentration, and ammonia concentration in a sample, such as saliva, by determining whether each of the examination items is positive or negative in a specific order, the stage of a periodontal disease can be objectively determined by a small number of steps and the determination result is highly reliable, and achieved the present invention.

More specifically, the present invention is as follows.

A first mode of the present invention is the following method for determining a stage of a periodontal disease (hereinafter, also referred to as a "determination method of the present invention").
[1] An *in vitro* method for determining the stage of a periodontal disease by using as an index at least two selected from the amount of occult blood, the number of white blood cells, and the total protein concentration in an examination object obtained from an oral cavity, the method including:
   a) a step of measuring at least two selected from the amount of occult blood, the number of white blood cells, and the total protein concentration in said examination object; and
   b) a step of determining whether the amount of occult blood or the total protein concentration is positive or negative; and
   c) a step of determining whether the number of white blood cells is positive or negative when the amount of occult blood or the total protein concentration is positive; or
   d) a step of determining whether the total protein concentration is positive or negative when the amount of occult blood is negative; or
   a step of determining whether the amount of occult blood is positive or negative when the total protein concentration is negative,
   wherein
   a case where the amount of occult blood is positive, and the number of white blood cells is positive is determined as serious periodontal infection,
   a case where the amount of occult blood is positive, and the number of white blood cells is negative is determined as intermediate periodontal infection,
   a case where the amount of occult blood is negative, and the total protein concentration is positive is determined as light periodontal infection,
   a case where the amount of occult blood is negative, and the total protein concentration is negative is determined as gingivitis,
   a case where the total protein concentration is positive, and the number of white blood cells is positive is determined as serious periodontal infection,
   a case where the total protein concentration is positive, and the number of white blood cells is negative is determined as light or intermediate periodontal infection,
   a case where the total protein concentration is negative, and the amount of occult blood is positive is determined as intermediate periodontal infection,
   a case where the total protein concentration is negative, and the amount of occult blood is negative is determined as gingivitis.
[2] The method according to [1], including:
   a) a step of determining whether the amount of occult blood is positive or negative; and
   b) a step of determining whether the number of white blood cells is positive or negative when the amount of occult blood is positive; or
   a step of determining whether the total protein concentration is positive or negative when the amount of occult blood is negative, in which
   a case where the amount of occult blood is positive, and the number of white blood cells is positive is determined as serious periodontal infection,
   a case where the amount of occult blood is positive, and the number of white blood cells is negative is determined as intermediate periodontal infection,
   a case where the amount of occult blood is negative, and the total protein concentration is positive is determined as light periodontal infection, and
   a case where the amount of occult blood is negative, and the total protein concentration is negative is determined as gingivitis.
[3] The method according to [1] or [2], further including:
   a step of using ammonia concentration as an index and determining whether the ammonia concentration is positive or negative.
[4] The method according to [3], in which
   a case where the amount of occult blood is positive, the number of white blood cells is positive, and the ammonia concentration is positive is determined as serious periodontal infection,
   a case where the amount of occult blood is positive, the number of white blood cells is positive, and the ammonia concentration is negative is determined as a periodontal disease improvement period,
   a case where the amount of occult blood is positive, the number of white blood cells is negative, and the ammonia concentration is positive is determined as intermediate periodontal infection,
   a case where the amount of occult blood is positive, the number of white blood cells is negative, and the ammonia concentration is negative is determined as the periodontal disease improvement period,
   a case where the amount of occult blood is negative, the total protein concentration is positive, and the ammonia concentration is positive is determined as light periodontal infection,
   a case where the amount of occult blood is negative, the total protein concentration is positive, and the ammonia concentration is negative is determined as the periodontal disease improvement period,
   a case where the amount of occult blood is negative, the total protein concentration is negative, and the ammonia concentration is positive is determined as gingivitis, and
   a case where the amount of occult blood is negative, the total protein concentration is negative, and the ammonia concentration is negative is determined as healthy.
   A second mode of the present invention is the following determination result sheet generation device of the stage of a periodontal disease and examination device including the same (hereinafter, also referred to as a "generation device of the present invention" and an "examination device of the present invention").
[5] A determination result sheet generation device including:
   an input unit configured to receive measurement values of the amount of occult blood, the number of white blood cells, the total protein concentration, and the ammonia concentration in the examination object obtained from the oral cavity;
   a determination unit configured to determine the stage of a periodontal disease by the method according to any one of [1] to [4] excluding step a) of [1];
   a display unit configured to display a determination result determined in the determination unit and a comment corresponding to the determination result;
   a storage unit configured to store the measurement values, the determination result, and the comment; and
   a control unit configured to generate a determination result sheet including the measurement values, the determination result, and the comment.
[6] The determination result sheet generation device according to [5], further including:
   a communication unit configured to output the generated determination result sheet to an external device.
[7] An examination device including:
   the determination result sheet generation device according to [5] or [6]; and
   a measurement unit configured to measure the amount of occult blood, the number of white blood cells, the total protein concentration, and the ammonia concentration in the examination object obtained from the oral cavity.

   A third mode of the present invention is the following determination result sheet generation method of the stage of a periodontal disease (hereinafter, also referred to as a "generation method of the present invention").
[8] A determination result sheet generation method in which a computer executes:
   a step of receiving measurement values of the amount of occult blood, the number of white blood cells, the total protein concentration, and the ammonia concentration in the examination object obtained from the oral cavity;
   a step of determining the stage of a periodontal disease by the method according to any one of [1] to [4] excluding step a) of [1];
   a step of selecting a comment corresponding to a determined determination result; and
   a step of generating a determination result sheet including the measurement values, the determination result, and the selected comment.
[9] The determination result sheet generation method according to [8], in which
   the computer further executes a step of outputting the generated determination result sheet to an external device.
   A fourth mode of the present invention is the following determination result sheet generation program of the stage of a periodontal disease (hereinafter, also referred to as a "program of the present invention").
[10] A determination result sheet generation program that makes a computer execute:
   a step of receiving measurement values of the amount of occult blood, the number of white blood cells, the total protein concentration, and the ammonia concentration in the examination object obtained from the oral cavity;
   a step of determining the stage of a periodontal disease by the method according to any one of [1] to [4] excluding step a) of [1];
   a step of selecting a comment corresponding to a determined determination result; and
   a step of generating a determination result sheet including the measurement values, the determination result, and the selected comment.
[11] The determination result sheet generation program according to [10], which makes the computer further execute a step of outputting the generated determination result sheet to an external device.

### Advantageous Effects of Invention

According to the determination method of the present invention, on the basis of a measurement result of examination items for an examination object obtained from an oral cavity, a highly-reliable determination result of a stage of a periodontal disease can be objectively obtained by an easy and quick procedure, two steps at the minimum, without intervention of a dentist or the like. In addition, according to the generation device, examination device, generation method, and program of the present invention, the highly-reliable determination result of the stage of a periodontal disease can also be provided to situations, such as a drugstore and a group medical examination, without limiting to a dental office, and the usability is high.

### Brief Description of Drawings

FIG. 1 is a diagram illustrating one mode of a determination flow of a stage of a periodontal disease;
FIG. 2 is a diagram illustrating one mode of the determination flow of the stage of a periodontal disease;
FIG. 3 is a diagram illustrating one mode of the determination flow of the stage of a periodontal disease;
FIG. 4 is a diagram illustrating a configuration example of a determination result sheet generation device;
FIG. 5 is a diagram illustrating a hardware configuration example of an information-processing device;
FIG. 6 is a diagram illustrating an operation flow example of generation of a determination result sheet by the determination result sheet generation device; and FIG. 7 is a diagram illustrating a flow of a type classification in examples.

### Description of Embodiments

Hereinafter, an embodiment will be described with reference to the drawings. A configuration of the embodiment is an example, and the present invention is not limited to a specific configuration of the disclosed embodiment. In embodying the present invention, a specific configuration corresponding to the embodiment may be appropriately adopted.

### <1> Method for Determining Stage of Periodontal Disease

In the present invention, the stage of a periodontal disease means the degree of progress of a periodontal disease expressed by multiple steps. Usually, the severity corresponding to an advanced stage is expressed in a stepwise fashion, and for example, the stage of a periodontal disease is expressed by gingivitis or periodontal infection, and more specifically, is preferably expressed by four stages, gingivitis, light periodontal infection, intermediate periodontal infection, and serious periodontal infection. In addition, further specifically, the stage of a periodontal disease is more preferably expressed by six stages, healthy, gingivitis, light periodontal infection, intermediate periodontal infection, serious periodontal infection, and an improvement period. Although only an independent state during an examination (for example, healthy to serious periodontal infection) is determined in a general determination of the degree of progress of an oral cavity disease, preferable one mode of the present invention is revolutionary in that a change from the state before the examination (improvement period) can also be determined. Generally, the improvement period can be considered as a healthy state as the stage of a periodontal disease.

Hereinafter, a general state of each of the stages will be described.

The "healthy" indicates a healthy state in which there are no periodontal disease bacteria, there is no bleeding from gingivae, and a depth of a gap of the boundary between teeth and gums (periodontal pocket) is about 1 to 2 mm.

The "gingivitis" indicates a state in which periodontal disease bacteria exist and gums start to become inflamed, but there is no bleeding from gingivae and a periodontal pocket is deepened to 2 to 3 mm.

The "light periodontal infection" indicates a state in which inflammation of gums is worsened by periodontal disease bacteria, and an alveolar bone and a periodontal membrane start to be broken. There are light symptoms in which there is bleeding from gingivae, or a periodontal pocket is deepened to 4 to 6 mm.

The "intermediate periodontal infection" indicates a state in which inflammation of gums is further worsened by periodontal disease bacteria, a breakdown of an alveolar bone progresses to nearly half, and teeth start to wobble. There are intermediate symptoms in which there is bleeding from gingivae, and a periodontal pocket is deepened to 4 to 6 mm.

The "serious periodontal infection" indicates a state in which more than half of an alveolar bone is broken by periodontal disease bacteria, and teeth wobble. There are serious symptoms in which there is bleeding from gingivae, and a periodontal pocket is deepened to 6 mm or more.

The "improvement period" indicates a state in which periodontal disease bacteria disappear from a mouth, and a periodontal disease goes in an improvement direction. Although a periodontal pocket is somewhat deep, 4 mm or more, getting better of symptoms, for example, there is no bleeding from gingivae, or the periodontal pocket is shallow, less than 4 mm, even if there is bleeding from the gingivae, is observed.

In the present invention, examples of an examination object obtained from an oral cavity include resting saliva (unstimulated saliva), stimulated saliva, and mouth wash discharge liquid. Examples of the stimulated saliva include saliva collected by stimulation with gum. Among these, the mouth wash discharge liquid is preferable. The mouth wash discharge liquid is obtained by, for example, taking purified water in a mouth and discharging it. Specifically, for example, 3 mL of purified water may be taken in the mouth for 10 seconds and discharged to a container. The volume of the purified water and time to be taken in the mouth can be appropriately changed as necessary. The obtained examination object can be used for a subsequent operation without special pretreatment, but an add operation, such as dilution, may be appropriately performed as necessary.

Examination items used as an index in the determination method of the present invention will be described.

The "amount of occult blood" means the amount of blood in the examination object obtained from the oral cavity. It is known that occult blood is detected in saliva when gum tissues are broken in association with a periodontal disease, and the amount of occult blood is an item that reflects the degree of breakage of periodontal tissues. As the amount of occult blood becomes larger, the degree of breakage of the periodontal tissues is higher and the stage of a periodontal disease tends to be more serious.

A measurement of the amount of occult blood is not particularly limited and can be performed by a known method. For example, preferably, the measurement is performed by a hemoglobin contact activation method. The hemoglobin contact activation method uses a fact that hemoglobin, myoglobin, or a decomposed product thereof as a blood component has an ability to catalyze oxygen transfer from an oxygen donor, such as peroxide, to an oxygen acceptor (peroxidase-like activity). An indicator that varies in shade due to oxidation is used as the oxygen acceptor, and the color reaction thereof is measured, so that the amount of occult blood is measured through a measurement of a concentration of hemoglobin or the like.

The "number of white blood cells" means the number of white blood cells in the examination object obtained from the oral cavity. It is known that white blood cells gather around a diseased area (inflamed area) of a periodontal disease, and the number of white blood cells is an item that reflects the degree of inflammation of periodontal tissues. As the number of white blood cells becomes larger, the degree of inflammation of the periodontal tissues is higher and the stage of a periodontal disease tends to be more serious.

A measurement of the number of white blood cells is not particularly limited and can be performed by a known method. For example, preferably, the measurement is performed by a white blood cell esterase method. The white blood cell esterase method uses a fact that white blood cells increase when tissues become inflamed, and esterase production by the white blood cells also increases in association therewith. The white blood cell esterase method is a method in which an ester compound is used as a substrate, and an alcohol (phenol) component generated by hydrolysis of the ester compound by esterase produced by white blood cells (white blood cell esterase) is directly made to be colored or the alcohol (phenol) is made to be colored by coupling with a diazonium salt, so that esterase activity is measured. The number of white blood cells can be calculated on the basis of the measurement value of the esterase activity.

A "total protein concentration" means a total protein concentration in the examination object obtained from the oral cavity. Since the total protein concentration becomes higher as the number of periodontal disease bacteria in the oral cavity becomes larger, the total protein concentration is an item that reflects the number of periodontal disease bacteria. As the total protein concentration becomes higher, the stage of a periodontal disease tends to be more serious.

A measurement of the total protein concentration is not particularly limited and can be performed by a known method. For example, preferably, the measurement is performed by a protein error method. The protein error method is a method using a fact that a pH indicator shows pH higher than real pH of a solution in proportion to a protein concentration, and the total protein concentration can be quantitated from coloring of the indicator.

An "ammonia concentration" means an ammonia concentration in the examination object obtained from the oral cavity. Since the ammonia concentration becomes higher as bacteria including periodontal disease bacteria grow more actively in the oral cavity, the ammonia concentration is an item that reflects the number of bacteria in the oral cavity. As the ammonia concentration becomes higher, the stage of a periodontal disease tends to be more serious.

Although the ammonia concentration is generally used as more an index of a determination of oral cavity cleanliness than a determination of a periodontal disease risk in a diagnosis of an oral cavity state, in a preferable mode of the determination method of the present invention, a more detailed and highly-reliable stage determination including the healthy state and the improvement period can be performed by using the ammonia concentration as an index in a determination of a periodontal disease risk.

A measurement of the ammonia concentration is not particularly limited and can be performed by a known method. For example, preferably, the measurement is performed by a microdiffusion method (Conway method). The microdiffusion method is a method used in quantification of ammonia nitrogen and is a method in which ammonia gas vaporized from a sample alkalified by an alkaline buffer is trapped in an absorbent solution or the like and the resulting coloring of an indicator is quantitated by a method, such as colorimetry.

A determination of whether each of the examination items is positive or negative is performed corresponding to the magnitude of the measurement value in each of the examination items. More specifically, in the case where the examination item is measured quantitatively, it is determined to be positive when the measurement value is larger and it is determined to be negative when the measurement value is smaller than a predetermined threshold value (cutoff value) determined in advance. In addition, the measurement of the examination item may be qualitative, and, also in this case, on the basis of a predetermined standard determined in advance, it is determined to be positive when the measurement result is larger and it is determined to be negative when the measurement result is smaller than the standard. It is to be noted that the cutoff value and the standard are set depending on the type of the examination object.

An example of the threshold value (cutoff value) when determining whether each of the examination items is positive or negative is shown in Table 1.

### [Table 1]

**Table 1: Example of Cutoff Value of Each of Examination Items**

| Degree of Risk | Negative | Positive |
|---|---|---|
| Amount of Occult Blood (mg/dL) | less than 0.01 | 0.01 or more |
| Number of White Blood Cells (U/L) | less than 4.5 | 4.5 or more |
| Total Protein Concentration (mg/dL) | less than 21 | 21 or more |
| Ammonia Concentration (µg/dL) | less than 1000 | 1000 or more |

The threshold value (cutoff value) when determining whether each of the examination items is positive or negative may be set by a value of an intermediate parameter in the measurement of the examination item in addition to the direct value, such as the concentration, as in Table 1. For example, when using the above-described color reaction in the measurement of the examination item, reflectivity data that correlates with the progress of the color reaction is obtained, and a reflectivity value may be used as the threshold value (cutoff value).

For example, the reflectivity data can be a reflectivity value at a specific wavelength after a lapse of a certain period of time since the examination object has been brought into contact with a measuring reagent of each parameter. Specifically, the reflectivity value can be obtained by irradiating a colored area, for example, an absorbent carrier area to which the examination object is applied with light at a specific wavelength and measuring the reflected light thereof. In addition, the reflectivity data may be a value obtained by subtracting the reflectivity value obtained as described above from 100%. It is to be noted that, as absorbance at a specific wavelength is higher, the reflectivity value at the specific wavelength is lower, and as absorbance at a specific wavelength is lower, the reflectivity value at the specific wavelength is higher. The certain period of time may be, for example, reaction time in each parameter measurement method as described above and may be appropriately set depending on the type and the measurement method of the parameter to be measured.

The determination method of the present invention is characterized by determining whether each of the examination items is positive or negative in a specific order by using as an index at least two selected from the amount of occult blood, the number of white blood cells, and the total protein concentration in the examination object obtained from the oral cavity.

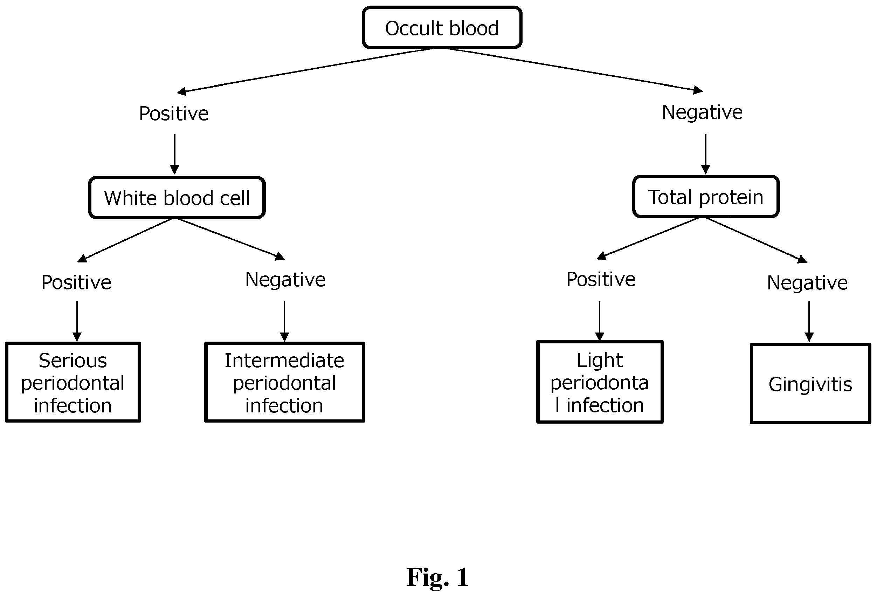

The determination method of the present invention is described with reference to a flow diagram illustrated in FIG. 1, and a step of determining whether the amount of occult blood is positive or negative is performed first in the determining steps. When the amount of occult blood is positive, a step of determining whether the number of white blood cells is positive or negative is performed, and when the amount of occult blood is negative, a step of determining whether the total protein concentration is positive or negative is performed. Four types classified through these steps correspond to states of the stage of a periodontal disease from serious to light in order of "the case where the amount of occult blood is positive, and the number of white blood cells is positive", "the case where the amount of occult blood is positive, and the number of white blood cells is negative", "the case where the amount of occult blood is negative, and the total protein concentration is positive", and "the case where the amount of occult blood is negative, and the total protein concentration is negative", and a result that reflects a real disease state is obtained.

Preferably, "the case where the amount of occult blood is positive, and the number of white blood cells is positive" is determined as the serious periodontal infection, "the case where the amount of occult blood is positive, and the number of white blood cells is negative" is determined as the intermediate periodontal infection, "the case where the amount of occult blood is negative, and the total protein concentration is positive" is determined as the light periodontal infection, and "the case where the amount of occult blood is negative, and the total protein concentration is negative" is determined as the gingivitis.

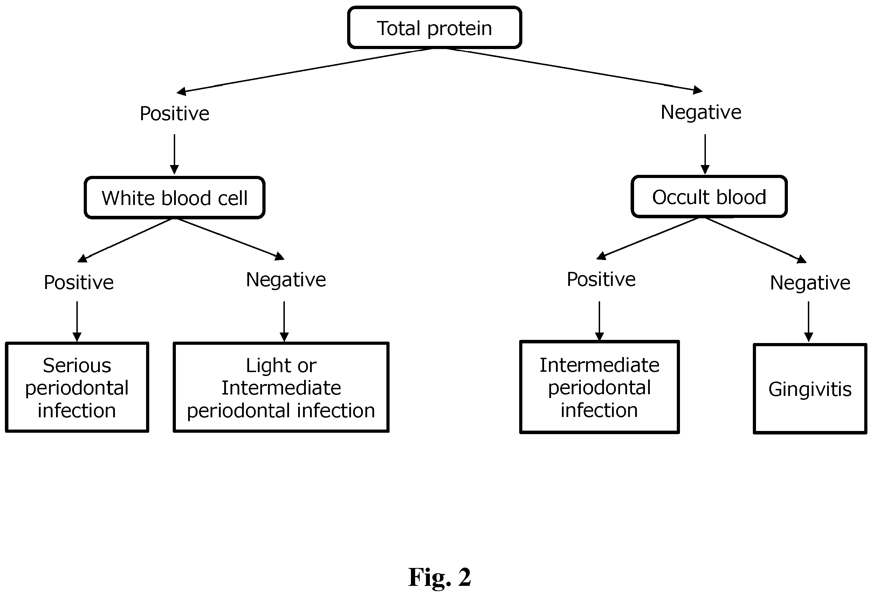

Another mode of the determination method of the present invention is described with reference to a flow diagram illustrated in FIG. 2, and a step of determining whether the total protein concentration is positive or negative is performed first in the determining steps. When the total protein concentration is positive, a step of determining whether the number of white blood cells is positive or negative is performed, and when the total protein concentration is negative, a step of determining whether the amount of occult blood is positive or negative is performed. These steps classify into four types, "the case where the total protein concentration is positive, and the number of white blood cells is positive", "the case where the total protein concentration is positive, and the number of white blood cells is negative", "the case where the total protein concentration is negative, and the amount of occult blood is positive", and "the case where the total protein concentration is negative, and the amount of occult blood is negative", and these are a result that reflects a real disease state.

Preferably, "the case where the total protein concentration is positive, and the number of white blood cells is positive" is determined as the serious periodontal infection, "the case where the total protein concentration is positive, and the number of white blood cells is negative" is determined as the light or intermediate periodontal infection, "the case where the total protein concentration is negative, and the amount of occult blood is positive" is determined as the intermediate periodontal infection, and "the case where the total protein concentration is negative, and the amount of occult blood is negative" is determined as the gingivitis.

It is important to perform the examination items (index) in which it is determined to be positive or negative and the order thereof as described above in the determination method of the present invention, it is necessary to measure the amount of occult blood or the total protein concentration in the first step, and preferably, the amount of occult blood is measured in the first step.

As shown in examples described below, the case where only the amount of occult blood is used as an index is insufficient as a determination of the stage of a periodontal disease (Table 4). In contrast, when performing the determination method of the present invention in which whether the number of white blood cells is positive or negative is determined when the amount of occult blood is positive and whether the total protein concentration is positive or negative is determined when the amount of occult blood is negative, the gingivitis, the light periodontal infection, the intermediate periodontal infection, and the serious periodontal infection can be significantly determined without determining whether all of the three items, the amount of occult blood, the number of white blood cells, and the total protein concentration are positive or negative (Table 5). In addition, when performing the determination method of the present invention in which whether the number of white blood cells is positive or negative is determined when the total protein concentration is positive and whether the amount of occult blood is positive or negative is determined when the total protein concentration is negative, a distinction between the gingivitis and the periodontal infection can be significantly determined, and furthermore, the gingivitis, the light or intermediate periodontal infection, the intermediate periodontal infection, and the serious periodontal infection can be determined without determining whether all of the three items, the amount of occult blood, the number of white blood cells, and the total protein concentration are positive or negative (Table 7).

Although it is generally considered that the reliability of a determination result is improved by increasing the number of parameters in the determination, according to the present invention, by using two examination items (index) that are the parameters used for the determination, which are appropriately selected and combined, ensuring of the reliability is achieved even in the determination based on the two parameters. In addition, the determination that can be performed in the two steps contributes to speeding-up and simplification.

It is to be noted that, even in the same two steps, in the case where whether the total protein concentration is positive or negative is determined when the amount of occult blood is positive and whether the number of white blood cells is positive or negative is determined when the amount of occult blood is negative (Table 6), in the case where whether the amount of occult blood is positive or negative is determined when the total protein concentration is positive and whether the number of white blood cells is positive or negative is determined when the total protein concentration is negative (Table 8), and in the case where the step of determining whether the number of white blood cells is positive or negative is performed first, and then, whether another item is positive or negative is determined (Tables 9, 10), the stage of a periodontal disease cannot be correctly determined.

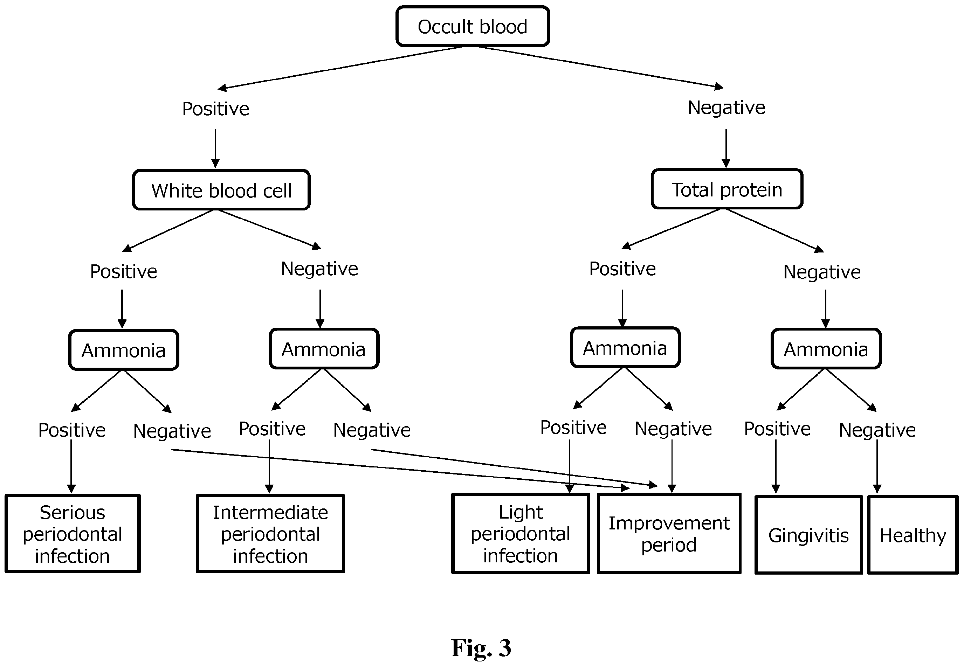

In the method of the present invention, preferably, a step of determining whether the ammonia concentration is positive or negative is further performed. Although not particularly limited, preferably, this step is added to the mode in which the step according to the amount of occult blood is performed first in the determining steps. This step may be performed before, between, or after the step according to the amount of occult blood or the total protein concentration and the step according to another item that is performed after the above step. More specifically, when the ammonia concentration is also used as an index, the step of determining whether the ammonia concentration is positive or negative is performed regardless of whether the other two items are positive or negative. It is to be noted that, although FIG. 3 illustrates an example of a determination flow of this mode, timing to perform the step of determining whether the ammonia concentration is positive or negative is not limited to the mode in which it is performed after the step according to the number of white blood cells or the total protein concentration as in FIG. 3.

Five types among eight types classified through these steps correspond to states of the stage of a periodontal disease from serious to light in order of "the case where the amount of occult blood is positive, the number of white blood cells is positive, and the ammonia concentration is positive", "the case where the amount of occult blood is positive, the number of white blood cells is negative, and the ammonia concentration is positive", "the case where the amount of occult blood is negative, the total protein concentration is positive, and the ammonia concentration is positive", "the case where the amount of occult blood is negative, the total protein concentration is negative, and the ammonia concentration is positive", and "the case where the amount of occult blood is negative, the total protein concentration is negative, and the ammonia concentration is negative", and a result that reflects a real disease state is obtained. Furthermore, three types, "the case where the amount of occult blood is positive, the number of white blood cells is positive, and the ammonia concentration is negative", "the case where the amount of occult blood is positive, the number of white blood cells is negative, and the ammonia concentration is negative" and "the case where the amount of occult blood is negative, the total protein concentration is positive, and the ammonia concentration is negative" correspond to an improving state of the periodontal disease and also reflect a real disease state.

Preferably, "the case where the amount of occult blood is positive, the number of white blood cells is positive, and the ammonia concentration is positive" is determined as the serious periodontal infection, "the case where the amount of occult blood is positive, the number of white blood cells is positive, and the ammonia concentration is negative" is determined as the periodontal disease improvement period, "the case where the amount of occult blood is positive, the number of white blood cells is negative, and the ammonia concentration is positive" is determined as the intermediate periodontal infection, "the case where the amount of occult blood is positive, the number of white blood cells is negative, and the ammonia concentration is negative" is determined as the periodontal disease improvement period, "the case where the amount of occult blood is negative, the total protein concentration is positive, and the ammonia concentration is positive" is determined as the light periodontal infection, "the case where the amount of occult blood is negative, the total protein concentration is positive, and the ammonia concentration is negative" is determined as the periodontal disease improvement period, "the case where the amount of occult blood is negative, the total protein concentration is negative, and the ammonia concentration is positive" is determined as the gingivitis, and "the case where the amount of occult blood is negative, the total protein concentration is negative, and the ammonia concentration is negative" is determined as the healthy.

By also using the ammonia concentration as an index, stages of the "healthy" and the "improvement period" can also be determined, and an objective, more detailed, and highly-reliable determination can be performed without intervention of a dentist or the like (refer to examples described below). In particular, it is revolutionary in that the "improvement period" that is a change from the state before the examination, which is not considered in the conventional general determination method, can also be determined.

### <2> Determination Result Sheet Generation Device, Generation Method, and Program of Stage of Periodontal Disease

A determination result sheet generation device of the stage of a periodontal disease of the present invention includes an input unit configured to receive measurement values of the amount of occult blood, the number of white blood cells, the total protein concentration, and the ammonia concentration in the examination object obtained from the oral cavity, a determination unit configured to determine the stage of a periodontal disease by the method of the present invention (excluding the measuring step), a display unit configured to display a determination result determined in the determination unit and a comment corresponding to the determination result, a storage unit configured to store the measurement values, the determination result, and the comment, and a control unit configured to generate a determination result sheet including the measurement values, the determination result, and the comment. Preferably, the determination result sheet generation device of the present invention further includes a communication unit configured to output the generated determination result sheet to an external device.

The determination result sheet generation device of the present invention is combined with a measurement unit configured to measure the amount of occult blood, the number of white blood cells, the total protein concentration, and the ammonia concentration in the examination object obtained from the oral cavity, so that a mode of an examination device can also be obtained.

Although not particularly limited and capable of being performed by a known method, as the measurement unit, one having a measuring means described in, for example, PTL 1 can be applied.

Hereinafter, a description will be provided with reference to the drawings.

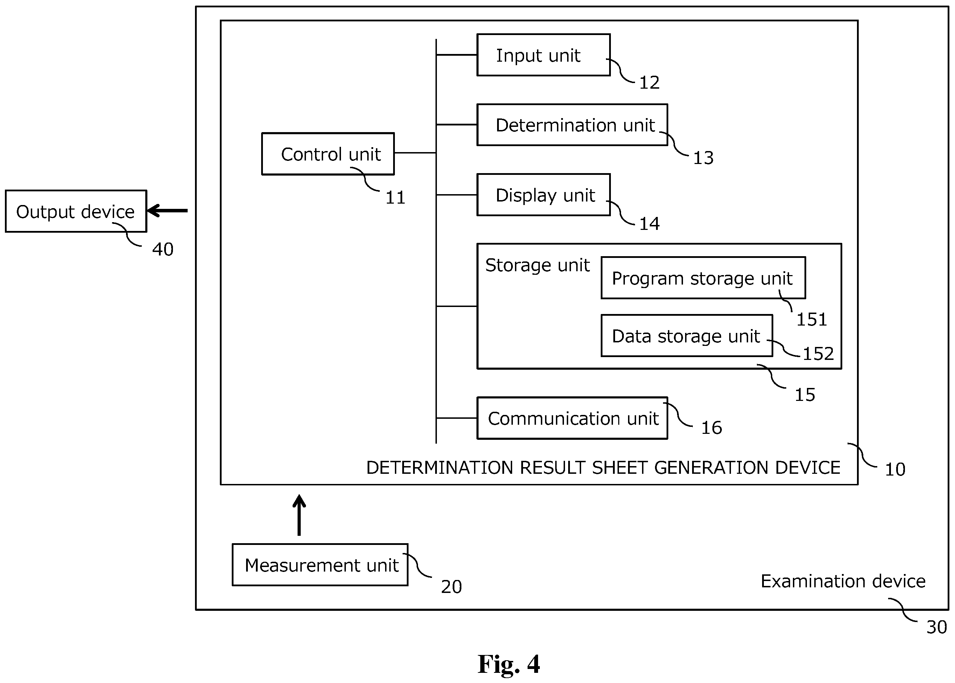

FIG. 4 illustrates a configuration example of a determination result sheet generation device 10 and an examination device 30 of the present invention. The determination result sheet generation device 10 includes a control unit 11, an input unit 12, a determination unit 13, a display unit 14, a storage unit 15, and a communication unit 16. The examination device 30 includes the determination result sheet generation device 10 and a measurement unit 20.

The measurement unit 20 measures the amount of occult blood, the number of white blood cells, the total protein concentration, and the ammonia concentration in the examination object obtained from the oral cavity of a subject. The determination result sheet generation device 10 obtains the measurement result from the measurement unit 20, determines the stage of a periodontal disease on the basis of this, and generates a determination result sheet.

The control unit 11 controls an entire operation of the determination result sheet generation device 10. The control unit 11 may control an operation of the measurement unit 20 via the communication unit 16.

The input unit 12 receives measurement values of the amount of occult blood, the number of white blood cells, the total protein concentration, and the ammonia concentration in the examination object obtained from the oral cavity from the measurement unit 20. The input unit 12 may receive the measurement values of the subject via a network or the like. In addition, the input unit 12 receives an input of characters and the like, and a selection, an operation, and the like of buttons and the like by a user (dentist, dental hygienist, person in charge of medical examination, drugstore clerk, subject himself/herself, and the like). The input unit 12 can be achieved by a keyboard, a pointing device, or the like.

The determination unit 13 determines the stage of a periodontal disease in accordance with the flow of the determination method of the present invention (excluding the measuring step) on the basis of the measurement values.

The storage unit 15 includes a program storage unit 151 and a data storage unit 152. The program storage unit 151 stores a determination result sheet generation program of the present invention. The data storage unit 152 stores data of the measurement values to be used in the determination unit 13, a determination result, a comment described below, subject information, and the like.

The display unit 14 displays the subject information, the measurement values, the determination result, the comment corresponding to the determination result, and the like. The display unit 14 can be achieved by, for example, a display device, such as a display.

The comment corresponding to the determination result is a message to be provided to the subject, such as a description of the determined stage of a periodontal disease, an instruction on a care method, or a proposal on a care product. For each determined stage of a periodontal disease, one comment corresponding to each item (the description of the stage of a periodontal disease, the instruction on a care method, or the proposal on a care product) is prepared in advance, and a corresponding comment is called and displayed on the basis of the determination result.

The communication unit 16 performs data communication between the measurement unit 20 and an output device 40, or the like. In addition, an external device, such as an external storage device, can be connected to the communication unit 16.

The output device 40 prints the determination result sheet including the subject information, the measurement values, the determination result, the comment corresponding to the determination result, and the like on paper or the like via the communication unit 16 on the basis of an instruction from the control unit 11. The output device 40 can be achieved by a printing device, such as a printer.

For example, by displaying an operation method of the measurement unit 20, the examination device 30 can make the user (dentist, dental hygienist, person in charge of medical examination, drugstore clerk, subject himself/herself, and the like) perform the collection of the examination object, such as saliva, and the measurement of the amount of occult blood, the number of white blood cells, the total protein concentration, and the ammonia concentration using the measurement unit 20. When the measurement unit 20 finishes the examination of the saliva, the determination result sheet generation device 10 receives in the input unit 12 and stores in the storage unit 15 the measurement values transmitted from the measurement unit 20.

The determination result sheet generation device 10 can be achieved by using a dedicated or general-purpose computer (information-processing device), such as a PC, a work station (WS), and a PDA (Personal Digital Assistant), or an electronic device equipped with a computer. In addition, the determination result sheet generation device 10 can also be achieved by using a smartphone, a mobile phone, a tablet type terminal, a dedicated or general-purpose computer, or an electronic device equipped with a computer.

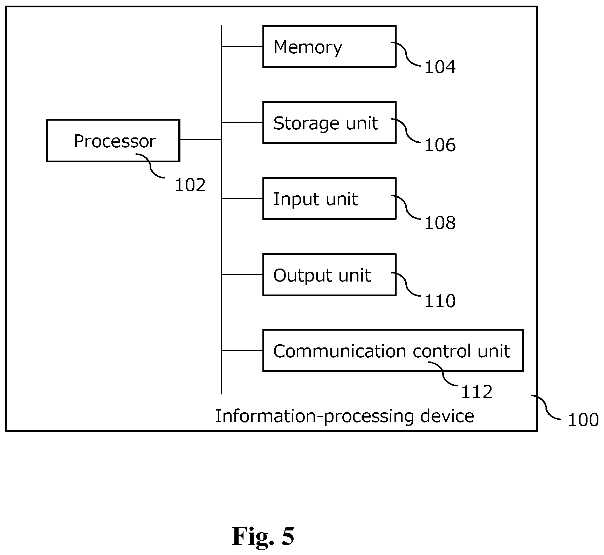

FIG. 5 illustrates a hardware configuration example of an information-processing device. The determination result sheet generation device 10 can be achieved by an information-processing device 100 as illustrated in FIG. 5. The information-processing device 100 of FIG. 5 has a processor 102, a memory 104, a storage unit 106, an input unit 108, an output unit 110, and a communication control unit 112. The memory 104 and the storage unit 106 are computer-readable recording media. The hardware configuration of an information-processing device is not limited to the example illustrated in FIG. 5, and omission, substitution, and addition of components may be appropriately performed.

The processor 102 loads a program stored in a recording medium into a work area of the memory 104 and executes the program, and the respective components and the like are controlled by the execution of the program, so that the information-processing device 100 can achieve functions consistent with predetermined purposes.

The processor 102 is, for example, a CPU (Central Processing Unit) or a DSP (Digital Signal Processor).

The memory 104 includes, for example, a RAM (Random Access Memory) or a ROM (Read Only Memory). The memory 104 is also referred to as a main storage device.

The storage unit 106 is, for example, an EPROM (Erasable Programmable ROM) or a hard disk drive (HDD). In addition, the storage unit 106 can include a removable medium, i.e. a portable recording medium. The removable medium is, for example, a USB (Universal Serial Bus) memory or a disc recording medium, such as a CD (Compact Disc) or a DVD (Digital Versatile Disc). The storage unit 106 is also referred to as a secondary storage device.

The storage unit 106 stores various programs, various pieces of data, and various tables in a recording medium freely readably and writably. An operating system (OS), various programs, various tables, and the like are stored in the storage unit 106. Information stored in the storage unit 106 may be stored in the memory 104. In addition, information stored in the memory 104 may be stored in the storage unit 106.

The operating system is software configured to perform mediation between software and hardware, management of memory space, file management, management of a process and a task, and the like. The operating system includes a communication interface. The communication interface is a program configured to exchange data with another external device or the like, which is connected via the communication control unit 112. Examples of the external device or the like include another information-processing device and an external storage device.

The input unit 108 includes a keyboard, a pointing device, a wireless remote controller, a touch panel, and the like. In addition, the input unit 108 can include an image or video input device, such as a camera, and a voice input device, such as a microphone.

The output unit 110 includes a display device, such as a CRT (Cathode Ray Tube) display, an LCD (Liquid Crystal Display), a PDP (Plasma Display Panel), and an EL (Electroluminescence) panel, and an output device, such as a printer. In addition, the output unit 110 can include a voice output device, such as a speaker.

The input unit 108 and the output unit 110 may be an input device and an output device that are peripheral devices independent of the information-processing device 100, respectively. In this case, the input device and the output device are connected to the information-processing device 100 via the communication control unit 112 of the information-processing device 100.

The communication control unit 112 is connected to another device and controls communication between the information-processing device 100 and the another device. The communication control unit 112 is, for example, a USB (Universal Serial Bus) interface board, a video output circuit, a LAN (Local Area Network) interface board, a wireless communication circuit for wireless communication, or a communication circuit for telephone communication. The LAN interface board or the wireless communication circuit is connected to a network, such as the Internet. The communication circuit for telephone communication is connected to a telephone communication network. For example, the information-processing device 100 is connected to the examination device 30 via the communication control unit 112.

The processor 102 loads a program stored in the secondary storage device into the main storage device and executes the program, so that the information-processing device configured to achieve the determination result sheet generation device 10 achieves functions of the control unit 11, the input unit 12, the determination unit 13, the display unit 14, and the communication unit 16. On the other hand, the storage unit 15 is provided in a storage area of the main storage device or the secondary storage device.

Each unit of the determination result sheet generation device 10 can be achieved as a component of hardware, a component of software, or a combination thereof.

The component of hardware is a hardware circuit, and examples thereof include a FPGA (Field Programmable Gate Array), an application specific integrated circuit (ASIC), a gate array, a combination of logic gates, and an analogue circuit.

The component of software is a part configured to achieve predetermined processing as software. The component of software is not a concept that limits language, a development environment, and the like configured to achieve software.

Steps that describe the program include not only processing to be performed in chronological order in the described order but also processing to be executed in parallel or individually even when not always being processed in chronological order. A part of the steps that describe the program may be omitted.

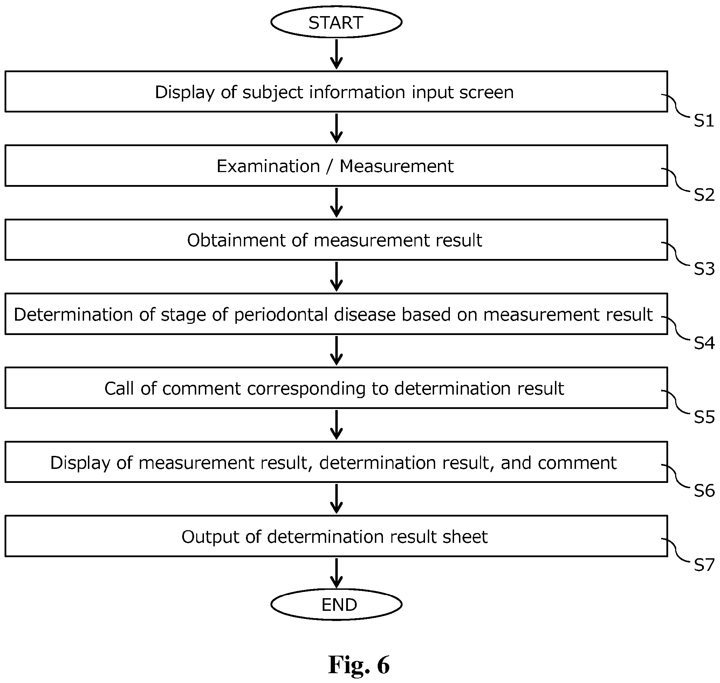

An operation example of the generation of the determination result sheet by the determination result sheet generation device 10 will be described with reference to FIG. 6. Here, an operation example of the determination result sheet generation device 10 when the user (dentist, dental hygienist, person in charge of medical examination, drugstore clerk, subject himself/herself, and the like) of the determination result sheet generation device 10 performs the examination for the subject using the measurement unit 20 and generates the determination result sheet is illustrated.

In Step S1, the control unit 11 of the determination result sheet generation device 10 displays on the display unit 14 a patient information input screen into which the subject information is to be inputted. The control unit 11 waits for input of the subject information by the user.

Although not limited thereto, examples of the subject information include name of patient, date of birth, sex, and medical record number.

When the subject underwent the examination in the past, a medical record table regarding the subject exists in the data storage unit 152. The medical record table is a table configured to store the subject information. One medical record table is generated for one subject, and each medical record table is identified by the medical record number. The medical record table of each subject can include a past examination result (measurement values, determination result, comment, and the like) in addition to the subject information.

When the medical record number of the subject is inputted by the user, the control unit 11 obtains from the data storage unit 152 the medical record table corresponding to the inputted medical record number, and extracts from the medical record table and uses as an item of the subject information to be displayed on the display unit 14 the name of the subject, the date of birth, the sex, and the like.

When the subject undergoes the examination for the first time, a medical record table regarding the subject does not exist in the data storage unit 152. Thus, the user inputs the subject information, such as the name of the subject, the date of birth, and the sex. The input unit 12 receives the subject information, and the control unit 11 newly generates a medical record table, registers the inputted subject information in the medical record table, and stores the medical record table in the data storage unit 152.

In Step S2, the control unit 11 of the determination result sheet generation device 10 displays an examination procedure on the display unit 14 and makes the measurement unit 20 measure the amount of occult blood, the number of white blood cells, the total protein concentration, and the ammonia concentration in the examination object obtained from the oral cavity of the subject via the communication unit 16.

The determination result sheet generation device 10 displays the examination procedure on the display unit 14. On the basis of the examination procedure or the like displayed on the display unit 14, the user transmits a signal indicating the start of the measurement to the determination result sheet generation device 10 from the measurement unit 20 after collecting the examination object from the oral cavity of the subject. Furthermore, the determination result sheet generation device 10 displays a measurement procedure or the like on the display unit 14. On the basis of the measurement procedure or the like displayed on the display unit 14, the user sets the examination object, for example, test paper to which the examination object is applied on the measurement unit 20 in an appropriate mode. In addition, for the set examination object, the determination result sheet generation device 10 makes the measurement unit 20 measure the amount of occult blood, the number of white blood cells, the total protein concentration, and the ammonia concentration. The measurement is performed by the measurement method described in, for example, PTL 1, and the measurement result is obtained as, for example, the reflectivity data that correlates with the progress of the color reaction. When the measurement is finished, a signal indicating the end of the measurement is transmitted to the determination result sheet generation device 10 from the measurement unit 20.

In Step S3, when receiving the signal indicating the end of the measurement from the measurement unit 20 via the communication unit 16, the control unit 11 of the determination result sheet generation device 10 instructs the measurement unit 20 to transmit the time and date of the examination and the measurement result. The measurement unit 20 transmits the time and date of the examination and the measurement result to the determination result sheet generation device 10. The measurement unit 20 may transmit the time and date of the examination and the measurement result to the determination result sheet generation device 10 together with the signal indicating the end of the measurement.

The determination result sheet generation device 10 receives in the input unit 12 and stores in the medical record table of the subject stored in the data storage unit 152 the time and date of the examination and the measurement result obtained from the measurement unit 20.

In Step S4, the control unit 11 of the determination result sheet generation device 10 makes the determination unit 13 determine the stage of a periodontal disease in accordance with the determination method of the present invention (excluding the measuring step) on the basis of the measurement result. The determination result is stored in the medical record table of the subject stored in the data storage unit 152.

In Step S5, the control unit 11 of the determination result sheet generation device 10 selects and calls a comment corresponding to the determination result from the data storage unit 152 and stores the comment in the medical record table of the subject stored in the data storage unit 152 as a result this time.

Regarding the comment corresponding to the determination result, for each determined stage of a periodontal disease, one comment is prepared in advance for the item, such as the description of the determined stage of a periodontal disease, the instruction on a care method, or the proposal on a care product. For example, when the determination result of the stage of a periodontal disease is the "light periodontal infection", a comment, such as the description of the stage of a periodontal disease, "In an initial stage of a periodontal disease. There are inflammation and bleeding in gums.", the instruction on a care method, "Please brush teeth with bristles of the toothbrush touched to the boundary between teeth and gingivae at an angle of 45°.", and the proposal on a care product, "Please use a dentifrice containing a bactericidal component.", is prepared and called in Step S5. The comment ensures that the appropriate description, the instruction, and the like corresponding to the determination result of the stage of a periodontal disease are objectively and automatically provided without intervention of a dentist or the like and can become helpful in self-medication of the subject.

In Step S6, the control unit 11 of the determination result sheet generation device 10 screen-displays the measurement result of the examination this time performed in Step S2, the determination result of the stage of a periodontal disease determined in Step S4, and the comment called in Step S5 on the display unit 14.

The display in this case may use only numbers and words, or diagrams (radar chart and the like).

In Step S7, the control unit 11 of the determination result sheet generation device 10 calls the examination result this time from the medical record table of the subject stored in the data storage unit 152, generates the determination result sheet including the subject information, the measurement result of the examination this time performed in Step S2, the determination result of the stage of a periodontal disease determined in Step S4, and the comment called in Step S5, and makes the output device 40 print the determination result sheet on paper. The printed determination result sheet is provided to the subject. The subject receives an explanation of the examination result and future care from the user.

In addition, the output in Step S7 may not be performed, and the user may make the explanation to the subject while viewing the determination result and the like displayed on the display unit 14 in Step S6.

### Examples

Hereinafter, the present invention will be described more specifically by examples, but the present invention is not limited to the examples.

### <1> Determination of Stage of Periodontal Disease by Dentist

The determination of the stage of a periodontal disease based on an oral cavity examination by a dentist was performed by the following procedure. More specifically, for 231 subjects who helped the examination, a probing examination by a dentist was performed, and a probing pocket depth (PD) and bleeding on probing (BOP) were evaluated. In both PD and BOP, six points were measured for one tooth, and a maximum PD of all teeth was calculated. In addition, the case where there was bleeding at only one site was evaluated as BOP (+), and the case where there was no bleeding at all teeth was evaluated as BOP (-). The stage of a periodontal disease was determined in accordance with Table 2 using these two clinical parameters.

### [Table 2]

**Table 2: Criteria for Determination of Stage of Periodontal Disease by Probing Examination**

| BOP | PD max | Stage of Periodontal Disease |
|---|---|---|
| (-) | less than 4 mm | Healthy |
| (-) | 4 mm or more | Improvement Period |
| (+) | less than 4 mm | Gingivitis |
| (+) | 4 mm or more and less than 5 mm | Light Periodontal Infection |
| (+) | 5 mm or more and less than 6 mm | Intermediate Periodontal Infection |
| (+) | 6 mm or more | Serious Periodontal Infection |

### <2> Measurement and Type Classification by Saliva Examination System

For the same 231 subjects as the above-described subjects, the measurement of each of the examination items by a saliva examination system was performed by the following procedure. More specifically, as the examination object, one obtained by taking 3 mL of purified water in the subject's mouth, lightly washing the mouth for 10 seconds, and then discharging it (mouth wash discharge liquid) was used. According to the method described in PTL 1, 10 µL of the examination object was applied to each absorbent carrier included in a test piece, and a salivary component was measured using PocketChem UA PU-4010 (manufactured by ARKRAY, Inc.). The measurement was performed at room temperature under the conditions of Table 3. A color change after a lapse of five minutes since the examination object had been applied was evaluated as the measurement value in each of the examination items by measuring a reflectivity (%) at a predetermined wavelength.

### [Table 3]

**Table 3: Measurement Condition of Each of Examination Items**

| Name of Measurement Item | Reaction Principle | Measurement Condition | | |
|---|---|---|---|---|
| | | Measurement Time (min) | Photometric Wavelength (nm) | Reference Wavelength (nm) |
| Amount of Occult Blood | Hemoglobin Contact Activation Method | 5 | 635 | - |
| Number of White Blood Cells | White Blood Cell Esterase Method | 5 | 565 | 760 |
| Total Protein Concentration | Protein Error Method | 5 | 635 | 760 |
| Ammonia Concentration | Microdiffusion Method | 5 | 635 | - |

Whether each of the examination items is positive or negative was determined by each cutoff value on the basis of the obtained reflectivity value. For the amount of occult blood, the case of 25% or more was determined to be negative, and the case of less than 25% was determined to be positive. For the number of white blood cells, the case of 60% or more was determined to be negative, and the case of less than 60% was determined to be positive. For the total protein concentration, the case of 55% or more was determined to be negative, and the case of less than 55% was determined to be positive. For the ammonia concentration, the case of 17% or more was determined to be negative, and the case of less than 17% was determined to be positive.

### <3> Concordance Rate between Type Classification by Saliva Examination System and Determination Result by Dentist

On the basis of whether each of the examination items is positive or negative, the respective subjects were classified into two types shown in Table 4, four types shown in each of Tables 5 to 10, or eight types shown in Table 11. Each table shows determination concordance rates between each type and the stages of a periodontal disease.

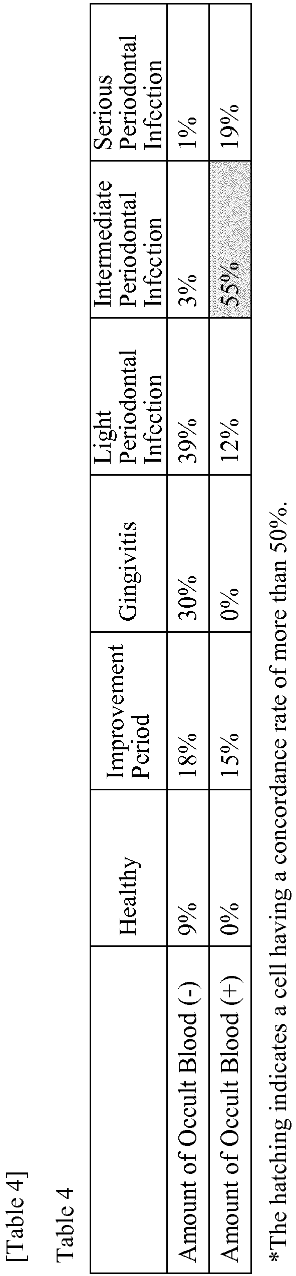

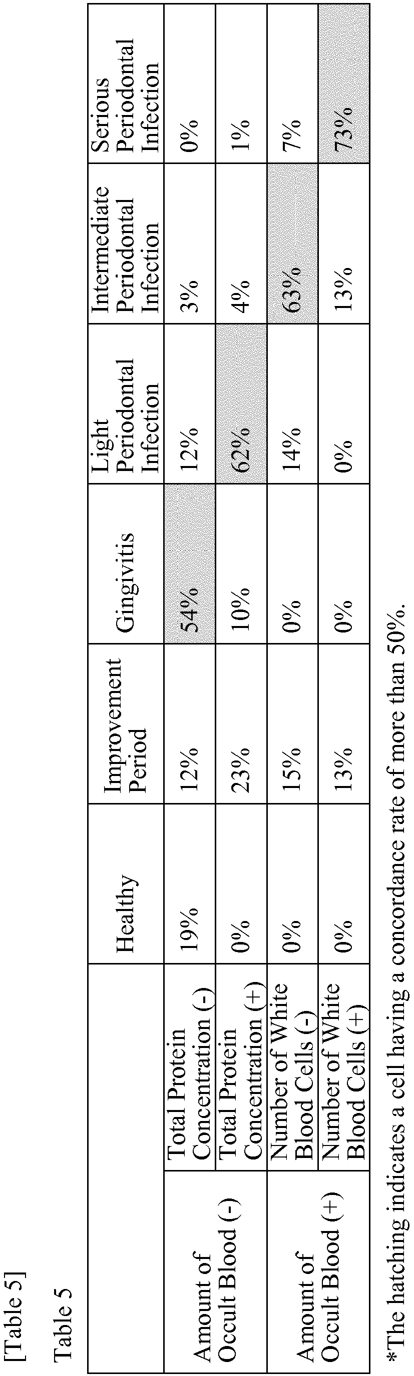

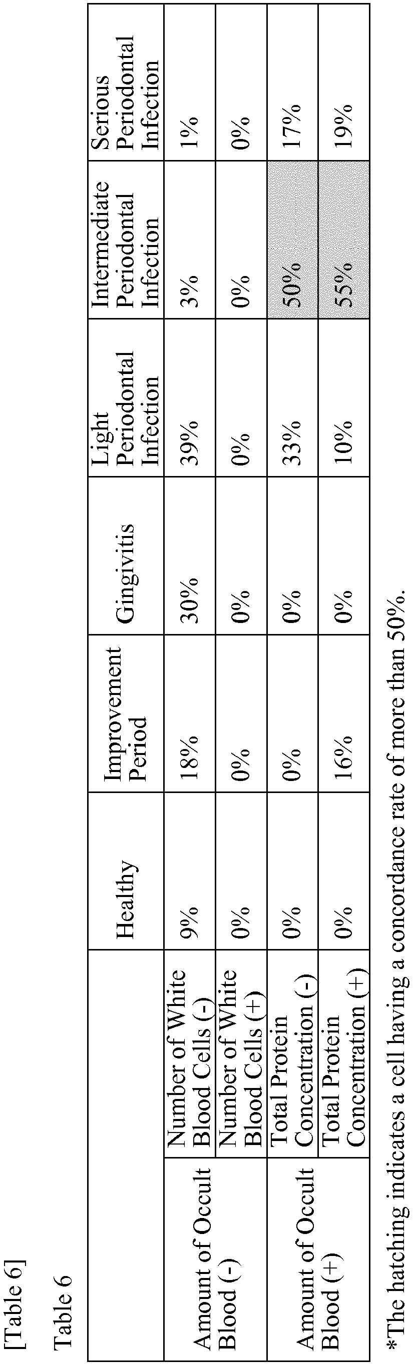

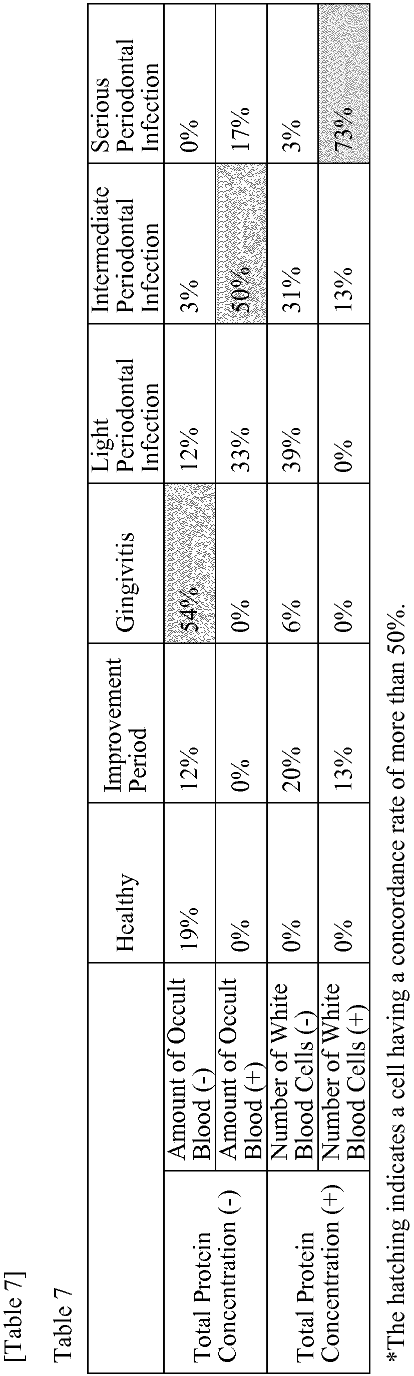

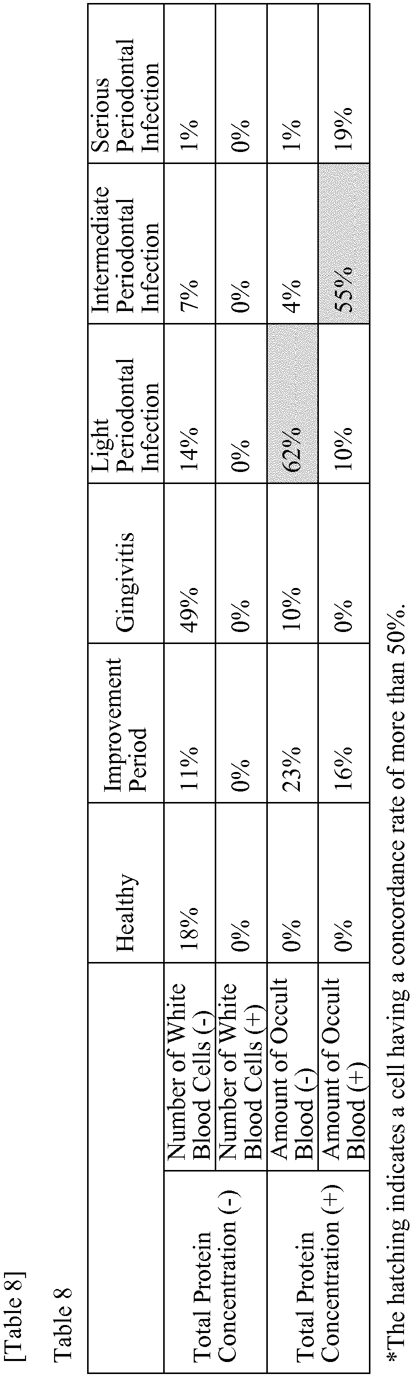

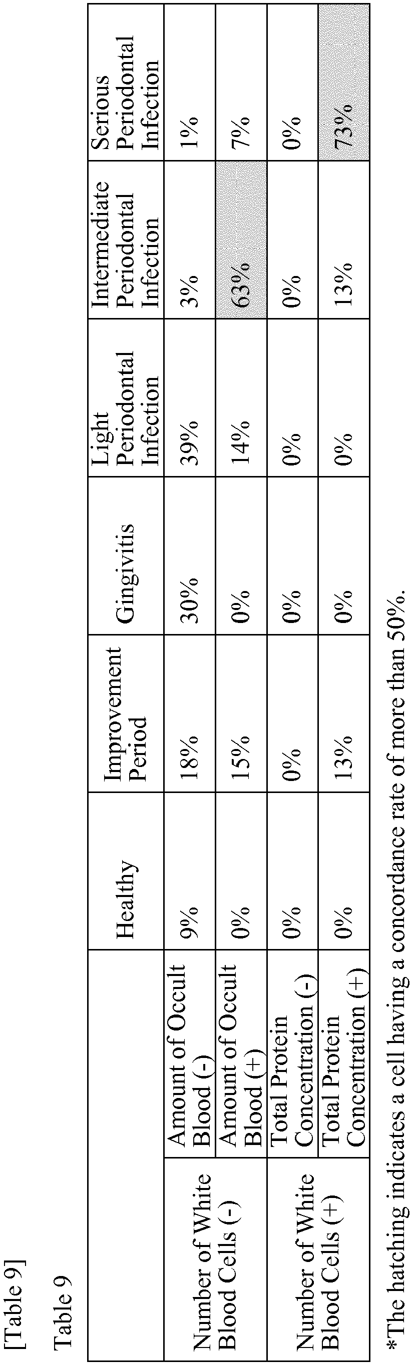

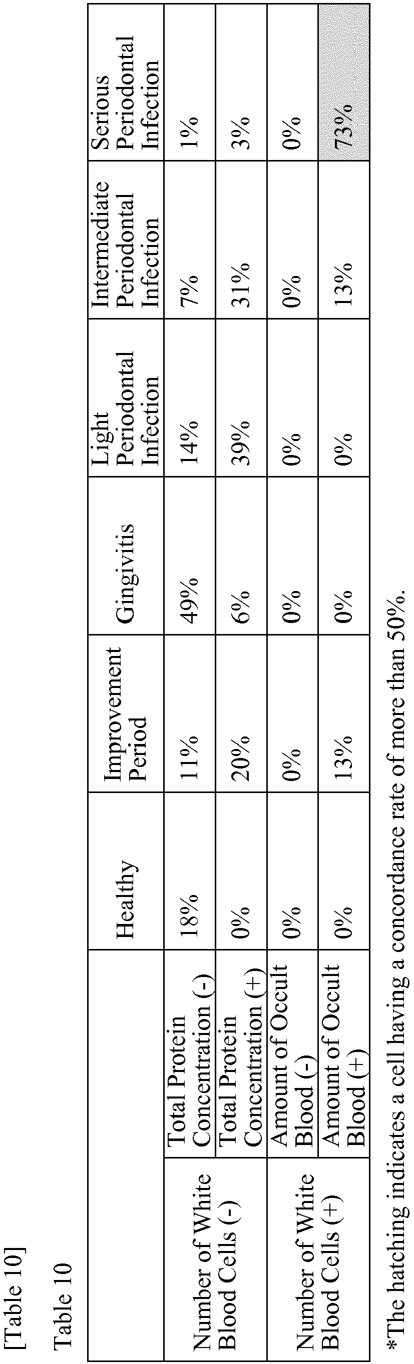

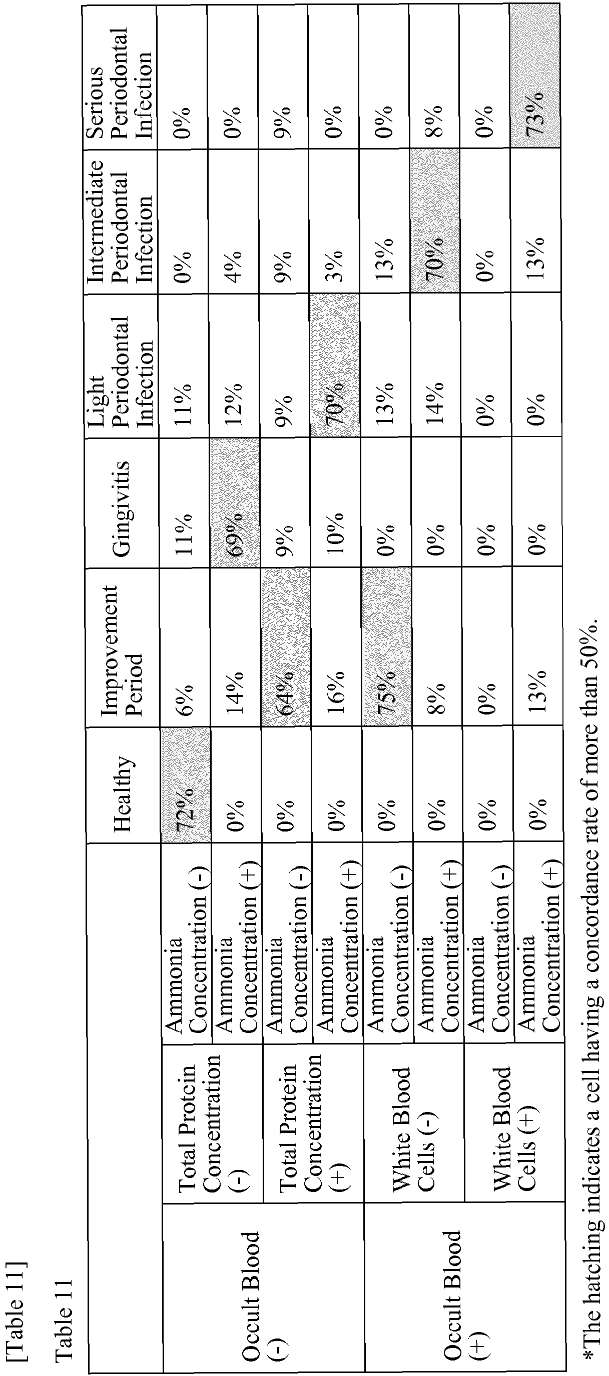

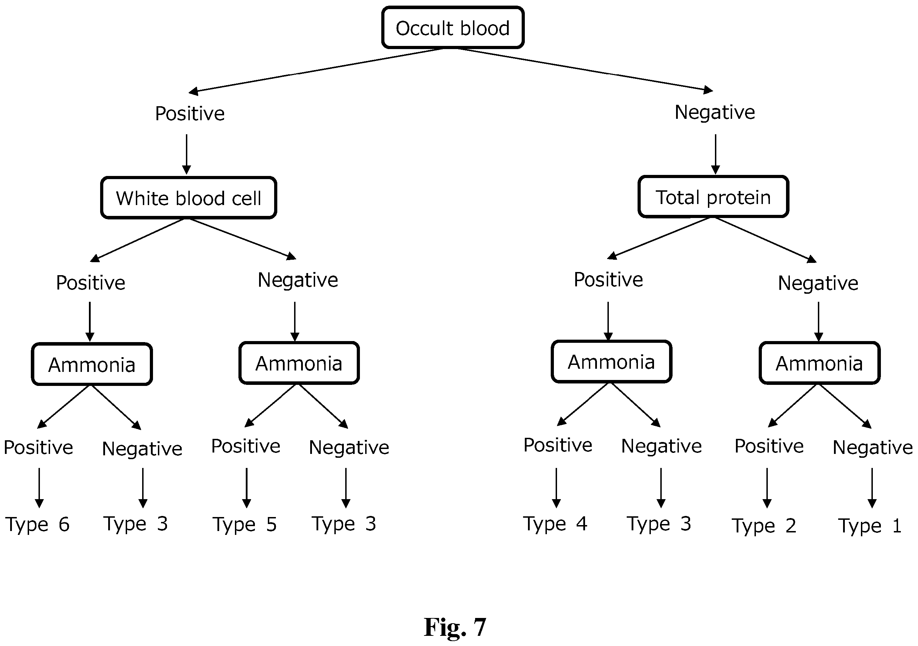

In addition, on the basis of whether each of the examination items is positive or negative when the reflectivity cutoff value was determined as 40% in the case of the amount of occult blood, 60% in the case of the number of white blood cells, 77% in the case of the total protein concentration, and 14% in the case of the ammonia concentration, the respective subjects were classified into six types in accordance with a flow of FIG. 7. Table 12 shows determination concordance rates between each type and the stages of a periodontal disease.

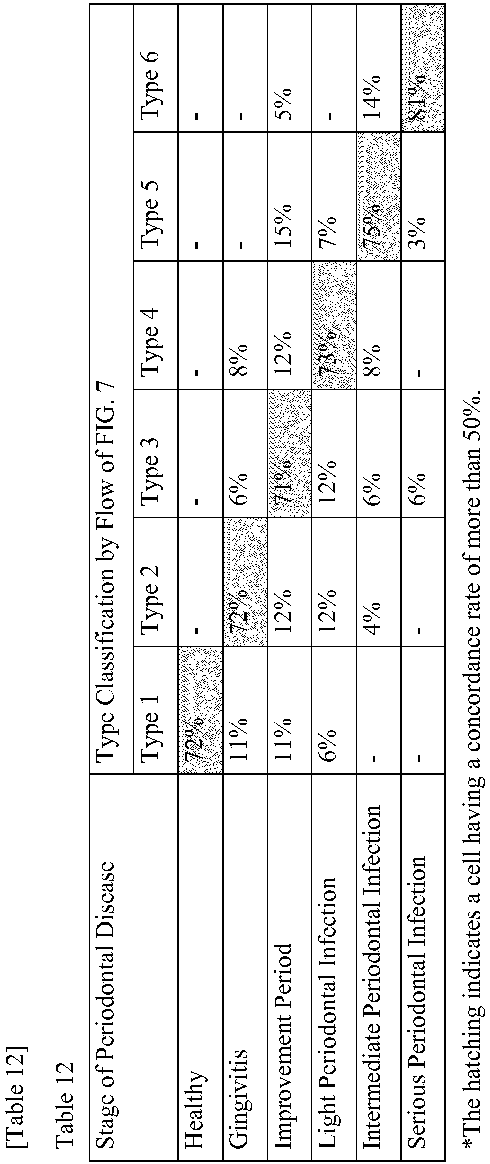

When the type classification was performed only by the amount of occult blood as the examination item, there were only two types, and thus, the correspondence between the stage of a periodontal disease and the determination by a dentist was insufficient (Table 4).

In the two-step type classification by three examination items, in the case where the type classification was performed by whether the number of white blood cells was positive or negative when the amount of occult blood was positive and the type classification was performed by whether the total protein concentration was positive or negative when the amount of occult blood was negative, one type classification corresponded to each of the stages of the gingivitis, the light periodontal infection, the intermediate periodontal infection, and the serious periodontal infection (Table 5). In the case where the type classification was performed by whether the number of white blood cells was positive or negative when the total protein concentration was positive and the type classification was performed by whether the amount of occult blood was positive or negative when the total protein concentration was negative, the determination by a dentist corresponded in the gingivitis and the periodontal infection, and thus, both can be clearly distinguished, and furthermore, the case where the total protein concentration was positive and the number of white blood cells was negative corresponded to the light or intermediate periodontal infection although the determination was not conclusive, and in other cases, the type classifications corresponded to the stages of the gingivitis, the intermediate periodontal infection, and the serious periodontal infection (Table 7). In the case where the type classification was performed by whether the total protein concentration was positive or negative when the amount of occult blood was positive and the type classification was performed by whether the number of white blood cells was positive or negative when the amount of occult blood was negative (Table 6), in the case where the type classification was performed by whether the amount of occult blood was positive or negative when the total protein concentration was positive and the type classification was performed by whether the number of white blood cells was positive or negative when the total protein concentration was negative (Table 8), and in the case where, after the type classification was performed by whether the number of white blood cells was positive or negative, the type classification was performed by whether another item was positive or negative (Tables 9, 10), the type classification corresponding to the stage of a periodontal disease was insufficient.

In the three-step type classification by four examination items, to which the ammonia concentration was also added, eight type classifications corresponded to more detailed stages, to which the stages of the healthy and the improvement period were added, including overlap (Tables 11, 12).

From these results, it is found that the determination method of the present invention can provide a highly-reliable determination result.

### Reference Signs List

10 determination result sheet generation device
11 control unit
12 input unit
13 determination unit
14 display unit
15 storage unit
151 program storage unit
152 data storage unit
16 communication unit
20 measurement unit
30 examination device
40 output device
100 information-processing device
102 processor
104 memory
106 storage unit
108 input unit
110 output unit
112 communication control unit

## Claims

1. An *in vitro* method for determining the stage of a periodontal disease by using as an index at least two selected from the amount of occult blood, the number of white blood cells, and the total protein concentration in an examination object obtained from an oral cavity, the method comprising:
a) a step of measuring at least two selected from the amount of occult blood, the number of white blood cells, and the total protein concentration in said examination object; and
b) a step of determining whether the amount of occult blood or the total protein concentration is positive or negative; and
c) a step of determining whether the number of white blood cells is positive or negative when the amount of occult blood or the total protein concentration is positive; or
d) a step of determining whether the total protein concentration is positive or negative when the amount of occult blood is negative; or
a step of determining whether the amount of occult blood is positive or negative when the total protein concentration is negative,
wherein
a case where the amount of occult blood is positive, and the number of white blood cells is positive is determined as serious periodontal infection,
a case where the amount of occult blood is positive, and the number of white blood cells is negative is determined as intermediate periodontal infection,
a case where the amount of occult blood is negative, and the total protein concentration is positive is determined as light periodontal infection,
a case where the amount of occult blood is negative, and the total protein concentration is negative is determined as gingivitis,
a case where the total protein concentration is positive, and the number of white blood cells is positive is determined as serious periodontal infection,
a case where the total protein concentration is positive, and the number of white blood cells is negative is determined as light or intermediate periodontal infection,
a case where the total protein concentration is negative, and the amount of occult blood is positive is determined as intermediate periodontal infection,
a case where the total protein concentration is negative, and the amount of occult blood is negative is determined as gingivitis.

2. The method according to claim 1, comprising:
a) a step of determining whether the amount of occult blood is positive or negative; and
b) a step of determining whether the number of white blood cells is positive or negative when the amount of occult blood is positive; or
a step of determining whether the total protein concentration is positive or negative when the amount of occult blood is negative,
wherein
a case where the amount of occult blood is positive, and the number of white blood cells is positive is determined as serious periodontal infection,
a case where the amount of occult blood is positive, and the number of white blood cells is negative is determined as intermediate periodontal infection,
a case where the amount of occult blood is negative, and the total protein concentration is positive is determined as light periodontal infection, and
a case where the amount of occult blood is negative, and the total protein concentration is negative is determined as gingivitis.

3. The method according to claim 1 or 2, further comprising:
a step of using ammonia concentration as an index and determining whether the ammonia concentration is positive or negative.

4. The method according to claim 3, wherein
a case where the amount of occult blood is positive, the number of white blood cells is positive, and the ammonia concentration is positive is determined as serious periodontal infection,
a case where the amount of occult blood is positive, the number of white blood cells is positive, and the ammonia concentration is negative is determined as a periodontal disease improvement period,
a case where the amount of occult blood is positive, the number of white blood cells is negative, and the ammonia concentration is positive is determined as intermediate periodontal infection,
a case where the amount of occult blood is positive, the number of white blood cells is negative, and the ammonia concentration is negative is determined as the periodontal disease improvement period,
a case where the amount of occult blood is negative, the total protein concentration is positive, and the ammonia concentration is positive is determined as light periodontal infection,
a case where the amount of occult blood is negative, the total protein concentration is positive, and the ammonia concentration is negative is determined as the periodontal disease improvement period,
a case where the amount of occult blood is negative, the total protein concentration is negative, and the ammonia concentration is positive is determined as gingivitis, and
a case where the amount of occult blood is negative, the total protein concentration is negative, and the ammonia concentration is negative is determined as healthy.

5. A determination result sheet generation device comprising:
an input unit configured to receive measurement values of the amount of occult blood, the number of white blood cells, the total protein concentration, and the ammonia concentration in the examination object obtained from the oral cavity;
a determination unit configured to determine the stage of a periodontal disease by the method according to any one of claims 1 to 4 excluding step a) of claim 1;
a display unit configured to display a determination result determined in the determination unit and a comment corresponding to the determination result;
a storage unit configured to store the measurement values, the determination result, and the comment; and
a control unit configured to generate a determination result sheet including the measurement values, the determination result, and the comment.

6. The determination result sheet generation device according to claim 5, further comprising:
a communication unit configured to output the generated determination result sheet to an external device.

7. An examination device comprising:
the determination result sheet generation device according to claim 5 or 6; and
a measurement unit configured to measure the amount of occult blood, the number of white blood cells, the total protein concentration, and the ammonia concentration in the examination object obtained from the oral cavity.

8. A determination result sheet generation method in which a computer executes:
a step of receiving measurement values of the amount of occult blood, the number of white blood cells, the total protein concentration, and the ammonia concentration in the examination object obtained from the oral cavity;
a step of determining the stage of a periodontal disease by the method according to any one of claims 1 to 4 excluding step a) of claim 1;
a step of selecting a comment corresponding to a determined determination result; and
a step of generating a determination result sheet including the measurement values, the determination result, and the selected comment.

9. The determination result sheet generation method according to claim 8, wherein
the computer further executes a step of outputting the generated determination result sheet to an external device.

10. A determination result sheet generation program that makes a computer execute:
a step of receiving measurement values of the amount of occult blood, the number of white blood cells, the total protein concentration, and the ammonia concentration in the examination object obtained from the oral cavity;
a step of determining the stage of a periodontal disease by the method according to any one of claims 1 to 4 excluding step a) of claim 1;
a step of selecting a comment corresponding to a determined determination result; and
a step of generating a determination result sheet including the measurement values, the determination result, and the selected comment.

11. The determination result sheet generation program according to claim 10, which makes the computer further execute a step of outputting the generated determination result sheet to an external device.

## Patentansprüche

1. *In-vitro*-Verfahren zum Ermitteln des Stadiums einer Parodontalerkrankung unter Verwendung mindestens zweier, ausgewählt aus der Menge okkulten Bluts, der Zahl weißer Blutzellen und der Gesamtproteinkonzentration in einem Untersuchungsobjekt, das aus einer Mundhöhle erhalten ist, als einen Index, wobei das Verfahren umfasst:
a) einen Schritt zum Messen mindestens zweier, ausgewählt aus der Menge okkulten Bluts, der Zahl weißer Blutzellen und der Gesamtproteinkonzentration im Untersuchungsobjekt; und
b) einen Schritt zum Ermitteln, ob die Menge okkulten Bluts oder die Gesamtproteinkonzentration positiv oder negativ ist; und
c) einen Schritt zum Ermitteln, ob die Zahl weißer Blutzellen positiv oder negativ ist, wenn die Menge okkulten Bluts oder die Gesamtproteinkonzentration positiv ist; oder
d) einen Schritt zum Ermitteln, ob die Gesamtproteinkonzentration positiv oder negativ ist, wenn die Menge okkulten Bluts negativ ist; oder
einen Schritt zum Ermitteln, ob die Menge okkulten Bluts positiv oder negativ ist, wenn die Gesamtproteinkonzentration negativ ist, wobei
ein Fall, in dem die Menge okkulten Bluts positiv ist und die Zahl weißer Blutzellen positiv ist, als schwere Parodontalinfektion ermittelt ist,
ein Fall, in dem die Menge okkulten Bluts positiv ist und die Zahl weißer Blutzellen negativ ist, als mäßige Parodontalinfektion ermittelt ist,
ein Fall, in dem die Menge okkulten Bluts negativ ist und die Gesamtproteinkonzentration positiv ist, als leichte Parodontalinfektion ermittelt ist,
ein Fall, in dem die Menge okkulten Bluts negativ ist und die Gesamtproteinkonzentration negativ ist, als Gingivitis ermittelt ist,
ein Fall, in dem die Gesamtproteinkonzentration positiv ist und die Zahl weißer Blutzellen positiv ist, als schwere Parodontalinfektion ermittelt ist,
ein Fall, in dem die Gesamtproteinkonzentration positiv ist und die Zahl weißer Blutzellen negativ ist, als leichte oder mäßige Parodontalinfektion ermittelt ist,
ein Fall, in dem die Gesamtproteinkonzentration negativ ist und die Menge okkulten Bluts positiv ist, als mäßige Parodontalinfektion ermittelt ist,
ein Fall, in dem die Gesamtproteinmenge negativ ist und die Menge okkulten Bluts negativ ist, als Gingivitis ermittelt ist.

2. Verfahren nach Anspruch 1, umfassend:
a) einen Schritt zum Ermitteln, ob die Menge okkulten Bluts positiv oder negativ ist; und
b) einen Schritt zum Ermitteln, ob die Zahl weißer Blutzellen positiv oder negativ ist, wenn die Menge okkulten Bluts positiv ist; oder
einen Schritt zum Ermitteln, ob die Gesamtproteinkonzentration positiv oder negativ ist, wenn die Menge okkulten Bluts negativ ist, wobei
ein Fall, in dem die Menge okkulten Bluts positiv ist und die Zahl weißer Blutzellen positiv ist, als schwere Parodontalinfektion ermittelt ist,
ein Fall, in dem die Menge okkulten Bluts positiv ist und die Zahl weißer Blutzellen negativ ist, als mäßige Parodontalinfektion ermittelt ist,
ein Fall, in dem die Menge okkulten Bluts negativ ist und die Gesamtproteinkonzentration positiv ist, als leichte Parodontalinfektion ermittelt ist, und
ein Fall, in dem die Menge okkulten Bluts negativ ist und die Gesamtproteinkonzentration negativ ist, als Gingivitis ermittelt ist.

3. Verfahren nach Anspruch 1 oder 2, weiter umfassend:
einen Schritt zum Verwenden von Ammoniakkonzentration als einen Index und Ermitteln, ob die Ammoniakkonzentration positiv oder negativ ist.

4. Verfahren nach Anspruch 3, wobei:
ein Fall, in dem die Menge okkulten Bluts positiv ist, die Zahl weißer Blutzellen positiv ist und die Ammoniakkonzentration positiv ist, als schwere Parodontalinfektion ermittelt ist,
ein Fall, in dem die Menge okkulten Bluts positiv ist, die Zahl weißer Blutzellen positiv ist und die Ammoniakkonzentration negativ ist, als eine Parodontalerkrankungsverbesserungsphase ermittelt ist,
ein Fall, in dem die Menge okkulten Bluts positiv ist, die Zahl weißer Blutzellen negativ ist und die Ammoniakkonzentration positiv ist, als mäßige Parodontalinfektion ermittelt ist,
ein Fall, in dem die Menge okkulten Bluts positiv ist, die Zahl weißer Blutzellen negativ ist und die Ammoniakkonzentration negativ ist, als die Parodontalerkrankungsverbesserungsphase ermittelt ist,
ein Fall, in dem die Menge okkulten Bluts negativ ist, die Gesamtproteinkonzentration positiv ist und die Ammoniakkonzentration positiv ist, als leichte Parodontalinfektion ermittelt ist,
ein Fall, in dem die Menge okkulten Bluts negativ ist, die Gesamtproteinkonzentration positiv ist und die Ammoniakkonzentration negativ ist, als die Parodontalerkrankungsverbesserungsphase ermittelt ist,
ein Fall, in dem die Menge okkulten Bluts negativ ist, die Gesamtproteinkonzentration negativ ist und die Ammoniakkonzentration positiv ist, als Gingivitis ermittelt ist, und
ein Fall, in dem die Menge okkulten Bluts negativ ist, die Gesamtproteinkonzentration negativ ist und die Ammoniakkonzentration negativ ist, als gesund ermittelt ist.

5. Ermittlungsergebnisbogenerstellungsvorrichtung, umfassend:
eine Eingabeeinheit, die konfiguriert ist, Messungswerte der Menge okkulten Bluts, der Zahl weißer Blutzellen, der Gesamtproteinkonzentration und der Ammoniakkonzentration in dem Untersuchungsobjekt, das aus der Mundhöhle erhalten ist, zu empfangen;
eine Ermittlungseinheit, die konfiguriert ist, das Stadium einer Parodontalerkrankung durch das Verfahren nach einem der Ansprüche 1 bis 4 ausschließlich Schritt a) von Anspruch 1 zu erhalten;
eine Anzeigeeinheit, die konfiguriert ist, ein Ermittlungsergebnis, das in der Ermittlungseinheit ermittelt ist, und einen Kommentar entsprechend dem Ermittlungsergebnis anzuzeigen;
eine Speichereinheit, die konfiguriert ist, die Messungswerte, das Ermittlungsergebnis und den Kommentar zu speichern; und
eine Steuerungseinheit, die konfiguriert ist, einen Ermittlungsergebnisbogen zu erstellen, der die Messungswerte, das Ermittlungsergebnis und den Kommentar beinhaltet.

6. Ermittlungsergebnisbogenerstellungsvorrichtung nach Anspruch 5, weiter umfassend:
eine Kommunikationseinheit, die konfiguriert ist, den erstellten Ermittlungsergebnisbogen an eine externe Vorrichtung auszugeben.

7. Untersuchungsvorrichtung, umfassend:
die Ermittlungsergebnisbogenerstellungsvorrichtung nach Anspruch 5 oder 6; und
eine Messungseinheit, die konfiguriert ist, die Menge okkulten Bluts, die Zahl weißer Blutzellen, die Gesamtproteinkonzentration und die Ammoniakkonzentration in dem Untersuchungsobjekt, das aus der Mundhöhle erhalten ist, zu messen.

8. Ermittlungsergebnisbogenerstellungsverfahren, in dem ein Computer ausführt:
einen Schritt zum Empfangen von Messungswerten der Menge okkulten Bluts, der Zahl weißer Blutzellen, der Gesamtproteinkonzentration und der Ammoniakkonzentration in dem Untersuchungsobjekt, das aus der Mundhöhle erhalten ist;
einen Schritt zum Ermitteln des Stadiums einer Parodontalerkrankung durch das Verfahren nach einem der Ansprüche 1 bis 4 ausschließlich Schritt a) von Anspruch 1;
einen Schritt zum Auswählen eines Kommentars entsprechend einem ermittelten Ermittlungsergebnis; und
einen Schritt zum Erstellen eines Ermittlungsergebnisbogens, der die Messungswerte, das Ermittlungsergebnis und den ausgewählten Kommentar beinhaltet.

9. Ermittlungsergebnisbogenerstellungsverfahren nach Anspruch 8, wobei
der Computer weiter einen Schritt zum Ausgeben des erstellten Ermittlungsergebnisbogens an eine externe Vorrichtung ausführt.

10. Ermittlungsergebnisbogenerstellungsprogramm, das einen Computer veranlasst auszuführen:
einen Schritt zum Empfangen von Messungswerten der Menge okkulten Bluts, der Zahl weißer Blutzellen, der Gesamtproteinkonzentration und der Ammoniakkonzentration in dem Untersuchungsobjekt, das aus der Mundhöhle erhalten ist;
einen Schritt zum Ermitteln des Stadiums einer Parodontalerkrankung durch das Verfahren nach einem der Ansprüche 1 bis 4 ausschließlich Schritt a) von Anspruch 1;
einen Schritt zum Auswählen eines Kommentars entsprechend einem ermittelten Ermittlungsergebnis; und
einen Schritt zum Erstellen eines Ermittlungsergebnisbogens, der die Messungswerte, das Ermittlungsergebnis und den ausgewählten Kommentar beinhaltet.

11. Ermittlungsergebnisbogenerstellungsprogramm nach Anspruch 10, das den Computer weiter veranlasst, einen Schritt zum Ausgeben des erstellten Ermittlungsergebnisbogens an eine externe Vorrichtung auszuführen.

## Revendications

1. Procédé *in vitro* de détermination du stade d'une maladie parodontale en utilisant comme indice au moins deux sélectionnés parmi la quantité de sang occulte, le nombre de globules blancs, et la concentration totale en protéines dans un objet d'examen obtenu à partir d'une cavité buccale, le procédé comprenant :
a) une étape consistant à mesurer au moins deux sélectionnés parmi la quantité de sang occulte, le nombre de globules blancs, et la concentration totale en protéines dans ledit objet d'examen ; et
b) une étape consistant à déterminer si la quantité de sang occulte ou la concentration totale en protéines est positive ou négative ; et
c) une étape consistant à déterminer si le nombre de globules blancs est positif ou négatif quand la quantité de sang occulte ou la concentration totale en protéines est positive ; ou
d) une étape consistant à déterminer si la concentration totale en protéines est positive ou négative quand la quantité de sang occulte est négative ; ou
une étape consistant à déterminer si la quantité de sang occulte est positive ou négative quand la concentration totale en protéines est négative,
dans lequel
un cas où la quantité de sang occulte est positive, et le nombre de globules blancs est positif est considéré comme une infection parodontale grave,
un cas où la quantité de sang occulte est positive, et le nombre de globules blancs est négatif est considéré comme une infection parodontale intermédiaire,
un cas où la quantité de sang occulte est négative, et la concentration totale en protéines est positive est considéré comme une infection parodontale légère,
un cas où la quantité de sang occulte est négative, et la concentration totale en protéines est négative est considéré comme une gingivite,
un cas où la concentration totale en protéines est positive, et le nombre de globules blancs est positif est considéré comme une infection parodontale grave,
un cas où la concentration totale en protéines est positive, et le nombre de globules blancs est négatif est considéré comme une infection parodontale légère ou intermédiaire,
un cas où la concentration totale en protéines est négative, et la quantité de sang occulte est positive est considéré comme une infection parodontale intermédiaire,
un cas où la concentration totale en protéines est négative, et la quantité de sang occulte est négative est considéré comme une gingivite.

2. Procédé selon la revendication 1, comprenant :
a) une étape consistant à déterminer si la quantité de sang occulte est positive ou négative ; et
b) une étape consistant à déterminer si le nombre de globules blancs est positif ou négatif quand la quantité de sang occulte est positive ; ou
une étape consistant à déterminer si la concentration totale en protéines est positive ou négative quand la quantité de sang occulte est négative,
dans lequel
un cas où la quantité de sang occulte est positive, et le nombre de globules blancs est positif est considéré comme une infection parodontale grave,
un cas où la quantité de sang occulte est positive, et le nombre de globules blancs est négatif est considéré comme une infection parodontale intermédiaire,
un cas où la quantité de sang occulte est négative, et la concentration totale en protéines est positive est considéré comme une infection parodontale légère, et
un cas où la quantité de sang occulte est négative, et la concentration totale en protéines est négative est considéré comme une gingivite.

3. Procédé selon la revendication 1 ou 2, comprenant en outre :
une étape consistant à utiliser une concentration en ammoniac comme indice et à déterminer si la concentration en ammoniac est positive ou négative.

4. Procédé selon la revendication 3, dans lequel
un cas où la quantité de sang occulte est positive, le nombre de globules blancs est positif, et la concentration en ammoniac est positive est considéré comme une infection parodontale grave,
un cas où la quantité de sang occulte est positive, le nombre de globules blancs est positif, et la concentration en ammoniac est négative est considéré comme une période d'amélioration d'une maladie parodontale,
un cas où la quantité de sang occulte est positive, le nombre de globules blancs est négatif, et la concentration en ammoniac est positive est considéré comme une infection parodontale intermédiaire,
un cas où la quantité de sang occulte est positive, le nombre de globules blancs est négatif, et la concentration en ammoniac est négative est considéré comme la période d'amélioration d'une maladie parodontale,
un cas où la quantité de sang occulte est négative, la concentration totale en protéines est positive, et la concentration en ammoniac est positive est considéré comme une infection parodontale légère,
un cas où la quantité de sang occulte est négative, la concentration totale en protéines est positive, et la concentration en ammoniac est négative est considéré comme la période d'amélioration d'une maladie parodontale,
un cas où la quantité de sang occulte est négative, la concentration totale en protéines est négative, et la concentration en ammoniac est positive est considéré comme une gingivite, et
un cas où la quantité de sang occulte est négative, la concentration totale en protéines est négative, et la concentration en ammoniac est négative est considéré comme sain.

5. Dispositif de génération de feuille de résultat de détermination comprenant :
une unité d'entrée configurée pour recevoir des valeurs de mesure de la quantité de sang occulte, du nombre de globules blancs, de la concentration totale en protéines, et de la concentration en ammoniac dans l'objet d'examen obtenu à partir de la cavité buccale ;
une unité de détermination configurée pour déterminer le stade d'une maladie parodontale par le procédé selon l'une quelconque des revendications 1 à 4 en excluant l'étape a) de la revendication 1 ;
une unité d'affichage configurée pour afficher un résultat de détermination déterminé dans l'unité de détermination et un commentaire correspondant au résultat de détermination ;
une unité de stockage configurée pour stocker les valeurs de mesure, le résultat de détermination, et le commentaire ; et
une unité de commande configurée pour générer une feuille de résultat de détermination incluant les valeurs de mesure, le résultat de détermination, et le commentaire.

6. Dispositif de génération de feuille de résultat de détermination selon la revendication 5, comprenant en outre :
une unité de communication configurée pour sortir la feuille de résultat de détermination générée vers un dispositif externe.

7. Dispositif d'examen comprenant :
le dispositif de génération de feuille de résultat de détermination selon la revendication 5 ou 6 ; et
une unité de mesure configurée pour mesurer la quantité de sang occulte, le nombre de globules blancs, la concentration totale en protéines, et la concentration en ammoniac dans l'objet d'examen obtenu à partir de la cavité buccale.

8. Procédé de génération de feuille de résultat de détermination dans lequel un ordinateur exécute :
une étape de réception de valeurs de mesure de la quantité de sang occulte, du nombre de globules blancs, de la concentration totale en protéines, et de la concentration en ammoniac dans l'objet d'examen obtenu à partir de la cavité buccale ;
une étape de détermination du stade d'une maladie parodontale par le procédé selon l'une quelconque des revendications 1 à 4 en excluant l'étape a) de la revendication 1 ;
une étape de sélection d'un commentaire correspondant à un résultat de détermination déterminé ; et
une étape de génération d'une feuille de résultat de détermination incluant les valeurs de mesure, le résultat de détermination, et le commentaire sélectionné.

9. Procédé de génération de feuille de résultat de détermination selon la revendication 8, dans lequel
l'ordinateur exécute en outre une étape de sortie de la feuille de résultat de détermination générée vers un dispositif externe.

10. Programme de génération de feuille de résultat de détermination qui amène un ordinateur à exécuter :
une étape de réception de valeurs de mesure de la quantité de sang occulte, du nombre de globules blancs, de la concentration totale en protéines, et de la concentration en ammoniac dans l'objet d'examen obtenu à partir de la cavité buccale ;
une étape de détermination du stade d'une maladie parodontale par le procédé selon l'une quelconque des revendications 1 à 4 en excluant l'étape a) de la revendication 1 ;
une étape de sélection d'un commentaire correspondant à un résultat de détermination déterminé ; et
une étape de génération d'une feuille de résultat de détermination incluant les valeurs de mesure, le résultat de détermination, et le commentaire sélectionné.

11. Programme de génération de feuille de résultat de détermination selon la revendication 10, qui amène l'ordinateur à exécuter en outre une étape de sortie de la feuille de résultat de détermination générée vers un dispositif externe.
